# EUROPEAN PATENT APPLICATION

(11) **EP 3 954 308 A1**
(43) Date of publication of application: **16.02.2022**
(21) Application number: 20786797.9
(22) Date of filing: 10.04.2020
(51) Int. Cl.: A61B 17/12

(54) **OCCLUDER, OCCLUDER FASTENING SYSTEM AND FASTENING METHOD**

(30) Priority: 11.04.2019 CN 201910291644; 11.04.2019 CN 201920491481 U
(71) Applicant: Hangzhou Endonom Medtech Co., Ltd, Hangzhou, Zhejiang 310051 (CN)
(72) Inventor: WANG, Yongsheng, Hangzhou, Zhejiang 310051 (CN); LIU, Xingzhi, Hangzhou, Zhejiang 310051 (CN)
(74) Representative: inCompass IP Europe Limited
(86) International application number: PCT/CN2020/084302
(87) International publication number: WO 2020/207484

(57) **Abstract**

Disclosed are an occluder (10), an occluder fastening system and a fastening method therefor. The occluder (10) includes a first occluding portion (11) and a second occluding portion (15). A distal end of the first occluding portion (11) is provided with a connecting bolt (112) extending towards the second occluding portion (15), and a first external thread (113) is provided on a side wall of the connecting bolt (112). A protrusion portion (152) is provided at a proximal end of the second occluding portion (15) with a through hole (153) provided therein, and a second external thread (154) is provided on a side wall of the protrusion portion (152). The thread direction of the first external thread (113) is opposite to the thread direction of the second external thread (154), and a proximal end of the connecting bolt (112) can extend through the through hole (153) and is screwed to a nut (689). In cooperation with a pushing device (60), such designed occluder (10) achieves high-efficiency, stable and reliable fastening, thereby improving the service life and occlusion performance of the occluder (10), reducing the risk of postoperative complications, which is particularly applicable for the occlusion treatment of rupture of aortic dissection.

## Description

### TECHNICAL FIELD

The present application relates to the technical field of medical devices, and in particular to an occluder, an occluder fastening system for delivering the occluder, and a fastening method for the occluder fastening system.

### BACKGROUND

A delivery system for an endoluminal occluder (such as left atrial appendage occluder, vascular plug, and filter, etc.) usually includes several parts such as a delivery sheath, an expansion device, a loading device, and a pushing device. A passage is established by the delivery sheath and the expansion device, and then the expansion device is withdrawn. The occluder is received in the loading device by means of the pushing device, and then the delivery sheath device and the loading device are connected. The occluder is advanced into the delivery sheath by the pushing device, until the occluder is delivered to a target location. With the development of transcatheter interventional treatment, various endoluminal occluders have been developed. However, the existing endoluminal occluders generally have problems such as poor reliability and convenience in the fastening process after having been released by a delivery device. This may affect the service life and the occlusion performance of the occluder, and increase the risk of complications.

### SUMMARY

In view of the shortcomings existing in the prior art, the present application provides an occluder, an occluder fastening system, and a fastening method for the occluder fastening system, with high fastening reliability and simple and convenient operations.

To solve the above technical problems, the present application provides an occluder, which includes a first occluding portion and a second occluding portion. A distal end of the first occluding portion is provided with a connecting bolt extending towards the second occluding portion, and a first external thread is provided on a side wall of the connecting bolt. A protrusion portion is provided at a proximal end of the second occluding portion, a through hole is provided in the protrusion portion, and a second external thread is provided on a side wall of the protrusion portion. The thread direction of the first external thread is opposite to the thread direction of the second external thread, and a proximal end of the connecting bolt can extend through the through hole and is screwed to a nut.

The present application also provides an occluder fastening system, which includes an occluder and a pushing device. The pushing device includes a first control component, a second control component and a pushing component. The pushing component includes a pushing tube and a pushing member mounted inside the pushing tube. An inner wall of the pushing tube at a distal end is provided with a second internal thread corresponding to the second external thread. The first control component can drive the pushing member to slidably move back and forth, to drive the proximal end of the connecting bolt which is detachably connected to the pushing member to abut against or move away from the nut. The first control component can drive the pushing tube to rotate, and the second control component can drive the pushing tube to rotate. The screwing direction between the first external thread and the nut is opposite to the screwing direction between the projection portion and the pushing tube.

The present application also provides a fastening method for an occluder fastening system, which includes a pre-fastening treatment, a fastening treatment and a post-fastening treatment. The pre-fastening treatment includes:

providing an occluder, a pushing device, and a sheath bendable adjustment device, wherein the occluder includes a nut, and a first occluding portion and a second occluding portion connected to each other, a distal end of the first occluding portion is provided with a connecting bolt and a proximal end of the second occluding portion is provided with a protrusion portion; the pushing device includes a pushing tube and a pushing member; and the sheath bendable adjustment device includes a sheath; and

engaging the nut with a distal end of the pushing tube, fitting the connecting bolt to the pushing member by threaded connection, fitting the protrusion portion to the pushing tube by threaded connection; and driving the pushing member to pull the occluder, then placing the occluder in the sheath and releasing the occluder.

The fastening treatment includes:
driving the pushing member to cause the connecting bolt to abut against the nut; and
rotating the pushing tube, so that the connecting bolt is threadedly connected to the nut; and in the meanwhile the pushing tube is disconnected from the occluder, and a proximal end of the connecting bolt is exposed from the nut.

The occluder provided in the present application includes a nut, a first occluding portion and a second occluding portion. The first occluding portion is provided with a connecting bolt and a first external thread is provided on a side wall of the connecting bolt. A protrusion portion is provided at the second occluding portion, a through hole is provided in the protrusion portion, and a second external thread is provided on a side wall of the protrusion portion. The thread direction of the first external thread is opposite to the thread direction of the first external thread, and a proximal end of the connecting bolt can extend through the through hole and is screwed to the nut. The occluder of the present application can be used in combination with a pushing device equipped with a pushing member and a pushing tube, to achieve fastening. Specifically, the pushing member can be detachably connected to the connecting bolt, and the pushing tube has an inner wall provided with an internal thread for connection with the protrusion portion and can accommodate the nut. The pushing device can control the pushing member to slide and rotate, and the pushing device can control the pushing tube to rotate. Because the thread direction of the first external thread is opposite to the thread direction of the second external thread, the pushing tube can be rotated to cause the connecting bolt to be screwed to the nut, and in the meanwhile cause the pushing tube to be disconnected from the protrusion portion. Therefore, when in cooperation with a pushing device, such a structure designed for the occluder of the present application can achieve high-efficiency, stable and reliable fastening, thereby improving the service life and occlusion performance of the occluder, reducing the risk of postoperative complications, which is particularly applicable for the occlusion treatment of rupture of aortic dissection.

### BRIEF DESCRIPTION OF THE DRAWINGS

In order to illustrate the technical solutions of the embodiments according to the present application more clearly, drawings used in the description of the embodiments according to the present application will be briefly introduced below. It should be appreciated that the drawings described below merely illustrate some embodiments of the present application, and other variations may be obtained by those skilled in the art without creative effort.
FIG. 1 is a structural schematic view of an occluder fastening system according to a first embodiment of the present application.
FIG. 2 is a structural schematic view of an occluder of the occluder fastening system shown in FIG. 1.
FIG. 3 is a schematic cross-sectional view of FIG. 2, taken along the line III-III.
FIG. 4 is a schematic exploded view of a pushing component of a pushing device of the occluder fastening system shown in FIG. 1.
FIG. 5 is a structural schematic view of a distal end surface of the pushing tube of the pushing component shown in FIG. 4.
FIG. 6 is a schematic cross-sectional view of FIG. 4, taken along the line III-III.
FIG. 7 is a schematic cross-sectional view of FIG. 4, showing a pushing member having been inserted into the pushing tube.
FIG. 8 is a structural schematic view of a sheath bendable adjustment device shown in FIG. 1.
FIG. 9 is a schematic cross-sectional view of FIG. 8, taken along the line IX-IX.
FIG. 10 is a structural schematic perspective view of a first shell shown in FIG. 8.
FIG. 11 is a structural schematic plan view of the first shell shown in FIG. 10.
FIG. 12 is a structural schematic perspective view of a second shell shown in FIG. 8.
FIG. 13 is a structural schematic plan view of the second shell shown in FIG. 12.
FIG. 14 is a schematic exploded view showing a distal cap shown in FIG. 8.
FIG. 15 is a structural schematic perspective view of a slipcover of the distal cap shown in FIG. 14.
FIG. 16 is a schematic cross-sectional view of FIG. 14, taken along the line XVI-XVI.
FIG. 17 is a structural schematic perspective view of a positioning tube shown in FIG. 8.
FIG. 18 is a schematic cross-sectional view of the positioning tube shown in FIG. 17.
FIG. 19 is a structural schematic perspective view of a proximal cap shown in FIG. 8.
FIG. 20 is a schematic cross-sectional view of the proximal cap shown in FIG. 19.
FIG. 21 is a structural schematic exploded view of an adjustment member and a driving member of an adjustment means shown in FIG. 8.
FIG. 22 is a schematic view of the adjustment member in FIG. 21, viewed from another aspect.
FIG. 23 is a schematic view of a sheath in FIG. 8.
FIG. 24 is a partially enlarged view of part XXIV in FIG. 23.
FIG. 25 is a schematic view of the sheath in FIG. 23, viewed from another aspect.
FIG. 26 is a cross-sectional view of FIG. 25, taken along the line XXVI-XXVI.
FIG. 27 is a partially enlarged view of part XXVII in FIG. 26.
FIG. 28 is a structural schematic view of the sheath bendable adjustment device shown in FIG. 8, with the second shell removed.
FIG. 29 is a structural schematic view of a pushing device for interventional instrument shown in FIG. 1.
FIG. 30 is a schematic exploded view of the pushing device for interventional instrument in FIG. 29.
FIG. 31 is a schematic cross-sectional view of FIG. 29, taken along the line XXXI-XXXI.
FIG. 32 is a structural schematic perspective exploded view of the first shell and the second shell in FIG. 30.
FIG. 33 is a structural schematic plan view showing an interior of the first shell in FIG. 32.
FIG. 34 is a structural schematic plan view showing an interior of the second shell in FIG. 32.
FIG. 35 is a structural schematic plan view showing an external of the second shell shown in FIG. 34.
FIG. 36 is a structural schematic perspective view of an outer buckle plate in FIG. 30.
FIG. 37 is a schematic exploded view of an end cap in FIG. 30.
] FIG. 38 is a cross-sectional view of FIG. 37, taken along the line XXXVIII-XXXVIII.
FIG. 39 is a structural schematic perspective exploded view showing a first clamping block, a movable block, and a second clamping block in FIG. 30.
FIG. 40 is a structural schematic view showing one side of the first clamping block in FIG. 39.
FIG. 41 is a structural schematic view showing the other side of the first clamping block in FIG. 39.
FIG. 42 is a structural schematic side view of the second clamping block in FIG. 39.
FIG. 43 is an enlarged view of the movable block in FIG. 39.
FIG. 44 is an enlarged view of a button in FIG. 30.
FIG. 45 is a structural schematic view of a first rotating means in FIG. 30.
FIG. 46 is a cross-sectional view of FIG. 45, taken along the line XLVI-XLVI.
FIG. 47 is a structural schematic perspective view of a rotating post in FIG. 45.
FIG. 48 is a structural schematic perspective view of a second rotating member in FIG. 30.
FIG. 49 is a schematic side view of the second rotating member in FIG. 48.
FIG. 50 is a cross-sectional view of FIG. 49, taken along the line L-L.
FIG. 51 is an assembled view of the pushing device in FIG. 30.
FIG. 52 is a cross-sectional view of FIG. 51, taken along the line LII-LII.
FIGs. 53 to 58 are schematic views illustrating the processes of fastening and releasing the occluder by the occluder fastening system in FIG. 3.
FIG. 59 is a structural schematic view of an occluder fastening system according to a second embodiment of the present application.
FIGs. 60 to 64 are schematic views illustrating the processes of fastening and releasing the occluder by the occluder fastening system in FIG. 59.
FIG. 65 is a structural schematic view of an occluder fastening system according to a third embodiment of the present application.
FIG. 66 is a schematic view illustrating the processes of fastening and releasing the occluder by the occluder fastening system in FIG. 65.
FIG. 67 is a flowchart of a fastening method of an occluder fastening system according to the present application.

### DESCRIPTION OF THE EMBODIMENTS

The technical solutions in the embodiments of the present application will be described clearly and fully in conjunction with the accompanying drawings in the embodiments of the present application. Apparently, the embodiments described are merely some, rather than all of the embodiments of the present application. Based on the embodiments of the present application, all other embodiments obtained by a person of ordinary skill in the art without creative efforts shall fall within the protection scope of the present application.

In addition, the following descriptions of various embodiments are provided to exemplify the specific embodiments of the present application with reference to accompanying drawings. The directional terms mentioned in the present application, such as "upper", "lower", "front", "back", "left", "right", "inner", "outer", and "side", etc., are only given with reference to the direction shown in the drawings. Therefore, the directional terms are used to explain more clearly and for better understanding of the present application, without indicating or implying that the device or element referred to requires a specific orientation, a construction and an operation in a specific orientation. Therefore, they cannot be understood as limitations to the present application. The term "axial direction" refers to the direction of the axis of the pushing member or the direction of the axis of the pushing tube.

Definitions for the orientations: for clarity of description, in the context with reference to the operation, the end close to the operator is referred to as "proximal end" and the end far away from the operator is referred to as "distal end". Axial direction refers to a direction parallel to a connection line between a distal center and a proximal center of an instrument. The above definitions are made for convenience of description only and shall not be construed as limit to the present application.

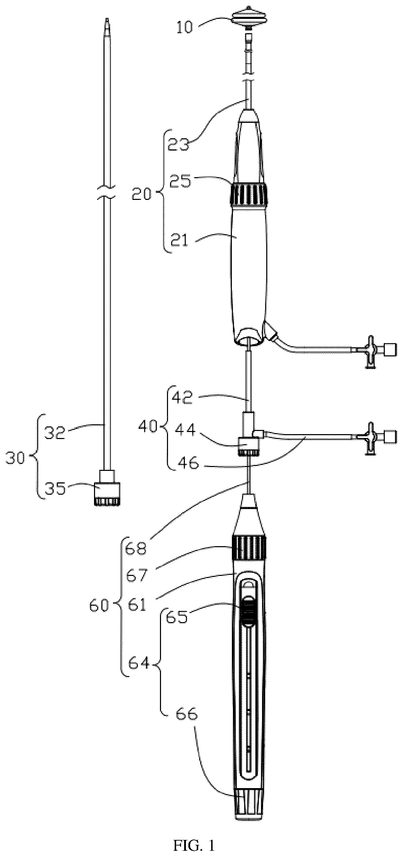

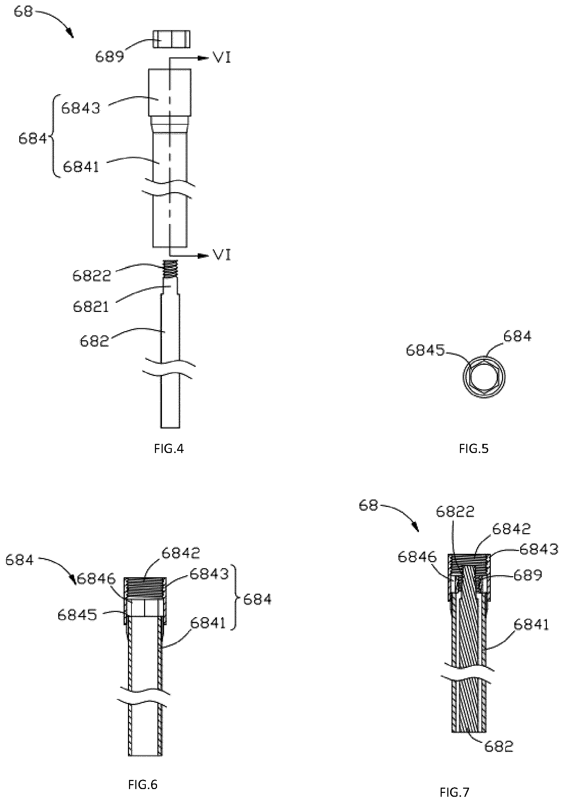

Referring to FIG. 1. the present application provides an occluder fastening system, which includes an occluder 10, a sheath bendable adjustment device 20, an expander 30, a loading component 40 disposed at a proximal end of the sheath bendable adjustment device 20, and a pushing device 60 disposed at a proximal end of the loading component 40. The sheath bendable adjustment device 20 includes a casing 21, a sheath 23 having a proximal end received in the casing 21, and an adjustment means 25 for bending a bendable distal end of the sheath 23. The loading component 40 includes a loading tube 42, a sealing seat 44 provided at a proximal end of the loading tube 42, and a three-way valve 46 radially communicating with the sealing seat 44. A distal end of the loading tube 42 can be inserted in the sheath 23. The pushing device 60 can push the occluder 10. The pushing device 60 includes a housing 61 with a receiving space, a first control component 64 partially received in the housing 61, a second control component 67 partially mounted around the housing 61, and a pushing component 68. The pushing component 68 includes a pushing member 682 (FIG 4). The pushing member 682 has an elongated structure, and a part of the pushing member 682 is received in the housing 61.

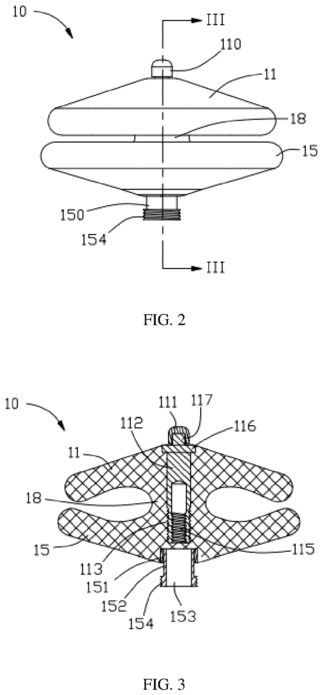

As shown in FIGs. 2 and 3, the occluder 10 includes a first occluding portion 11, a second occluding portion 15, and a waist 18 located between and connected with both the first occluding portion 11 and the second occluding portion 15. A distal end of the first occluding portion 11 is provided with a connecting bolt 112 extending towards the second occluding portion 15. That is, the connecting bolt 112 extends in an axial direction towards the second occluding portion 15. A side wall of the connecting bolt 112 is provided with a first external thread 113. A protrusion portion 152 is provided at a proximal end of the second occluding portion 15, and a through hole 153 is provided in the protrusion portion 152. Specifically, the through hole 153 is provided in the protrusion portion 152 along the axis of the occluder 10 and penetrates proximal and distal end surfaces of the protrusion portion 152. A side wall of the protrusion portion 152 is provided with a second external thread 154. The thread direction of the first external thread 113 is opposite to the thread direction of the second external thread 154. That is, the screwing direction of the first external thread 113 is opposite to the screwing direction of the second external thread 154. A proximal end of the connecting bolt 112 can extend through the through hole 153 and be screwed to a nut. The nut abuts against the proximal end surface of the protrusion portion 152. An effective thread length of the connecting bolt 112 is greater than or equal to an effective thread length of the nut. A proximal end surface of the connecting bolt 112 is provided with a screw hole 115 which extends axially, through which the connecting bolt 112 is detachably connected to a distal end of the pushing member 682. Specifically, an inner wall of the screw hole 115 is provided with a first internal thread, and the distal end of the pushing member 682 is screwed to the first internal thread.

As shown in FIG. 1, FIG. 3, and FIGs. 4 to 7, the pushing component 68 includes a pushing member 682, a pushing tube 684 movably mounted around the pushing member 682, and a nut 689 positioned inside a distal end of the pushing tube 684. A distal end of the pushing member 682 is detachably connected to the connecting bolt 112 of the occluder 10. An inner wall of the pushing tube 684 at a distal end thereof is provided with a second internal thread 6842 corresponding to the second external thread 154 of the protrusion portion 152. The first control component 64 can drive the pushing member 682 to axially slide back and forth in the pushing tube 684, to drive the connecting bolt 112 to abut against or move away from the screw hole of the nut 689. The second control component 67 includes a rotating means that can drive the pushing tube 684 to rotate, and thus the nut 689 is driven to rotate so that the connecting bolt 112 is screwed to the nut 689. Since the screwing direction between the connecting bolt 112 and the nut 689 is opposite to the screwing direction between the second external thread 154 of the protrusion portion 152 and the second internal thread 6842 of the pushing tube 684, during the process of screwing the connecting bolt 112 into the nut 689, the second external thread 154 of the protrusion portion 152 is disengaged from the second internal thread 6842 of the pushing tube 684.

The occluder 10 of the occluder fastening system in the present application includes the connecting bolt 112 provided at the distal end of the second occluding portion 15 and the protrusion portion 152 provided at the proximal end of the second occluding portion 15. The first external thread 113 is provided on the side wall of the connecting bolt 112, the second external thread 154 is provided on the side wall of the protrusion portion 152, and the thread direction of the first external thread 113 is opposite to the thread direction of the second external thread 154. The pushing device 60 of the occluder fastening system includes the first control component 64, the second control component 67 and the pushing component 68. The pushing component 68 includes the pushing member 682 and the pushing tube 684. The distal end of the pushing member 682 is detachably connected to the proximal end of the connecting bolt 112. The inner wall of the pushing tube 684 at the distal end is provided with the second internal thread 6842 corresponding to the second external thread 154. The first control component 64 can drive the pushing member 682 to slide axially, so as to drive the proximal end of the connecting bolt 112 to abut against or move away from the nut 689. The second control component 67 can drive the pushing tube 684 to rotate. Since the screwing direction between the connecting bolt 112 and the nut 689 is opposite to the screwing direction between the protrusion portion 152 and the pushing tube 684, therefore, when the connecting bolt 112 is screwed to the nut 689, the protrusion portion 152 is disengaged from the pushing tube 684.

In the occluder fastening system provided in the present application, since the proximal end of the connecting bolt 112 is fixedly connected to the nut 689 and the nut 689 abuts against the proximal end surface of the construction portion 152 when the occluder 10 is released, the occluder 10 can be fastened to a target position (such as a rupture) by screwing the nut 689 on the connecting bolt 112 after the occluder 10 is released, which facilitates the occluder 10 to maintain its axial length, thereby well maintaining the axial and radial dimensions and good occlusion state to prevent the occluder 10 from deforming or even migrating from the lesion site with the impact of blood flow. Therefore, the occluder 10 in the present application can be fastened with high reliability, thereby improving the occlusion performance of the occluder 10, reducing the risk of complications, and extending the service life of the occluder 10. In addition, when the proximal end of the connecting bolt 112 is screwed into the nut 689, the second external thread 154 of the protrusion portion 152 is disengaged from the second internal thread 6842 of the pushing tube 684, that is, the proximal end of the occluder 10 is disengaged from the pushing tube 684. This simplifies the detaching process of the occluder 10 from the pushing device 60. In the occluder fastening system, by operating the pushing device 60, the occluder 10 can be locked at the target position and the occluder 10 can be detached from the pushing tube 684 at the same time, which is convenient and simple to operate.

Specifically, the distal end of the first occluding portion 11 is provided with a first converged member 110, the proximal end of the second occluding portion 15 is provided with a second converged member 150. The first converged member 110 and the second converged member 150 are located in the axial direction of the occluder 10. The first converged member 110 includes an outer sleeve 111 disposed at the distal end of the first occluding portion 11 and the connecting bolt 112 connected to the outer sleeve 111. The second converged member 150 includes an inner sleeve 151 disposed at the proximal end of the second occluding portion 15 and the protrusion portion 152 connected to the inner sleeve 151. The first occluding portion 11 and the second occluding portion 15 are woven with metal wires and have a two-layer disc-shaped arc-surface structure with an internal space. The first occluding portion 11 and the second occluding portion 15 each may be provided with an occlusion membrane therein. The distal ends of the metal wires are converged and positioned between the outer sleeve 111 and the distal end of the connecting bolt 112, The proximal ends of the metal wires are converged and positioned between the inner sleeve 151 and the protrusion portion 152. The metal wires between the outer sleeve 111 and the connecting bolt 112 can be fixed by welding or clamping. Specifically, the outer sleeve 111 may be a cylinder with two open ends and the distal end of the metal wires are welded to an inner side of the cylinder. The distal end of the connecting bolt 112 is provided with a protruding surface extending radially, which has a diameter larger than the diameter of the cylinder. The proximal end of the connecting bolt 112 extends through the cylinder and the protruding surface is engaged to a distal end surface of the cylinder. In addition, the outer sleeve 111 may also have a structure with an inner cavity extending in a direction from the proximal end to the distal end, with the distal end of the connecting bolt 112 being fixed in the inner cavity, and the metal wires are welded at a position between the outer sleeve 111 and the connecting bolt 112. In this embodiment, the latter structure is employed. The metal wires arranged between the inner sleeve 151 and the protrusion portion 152 may also be fixed by welding, clamping, or other means. The distal end of the connecting bolt 112 is provided with a positioning disc 116 protruding radially, which may be annular, or square, etc. A distal end surface of the positioning disc 116 is provided with a post 117 protruding radially. During the fastening process of the occluder 10 (that is, when the proximal end of the connecting bolt 112 is screwed into the nut 689), the connecting bolt 112 is pulled towards the proximal end. If the force applied to the connecting bolt 112 is too large, the first converged member 110 woven with metal wires fixed to the connecting bolt 112 may be pulled into a rupture at the target site, which may make a circumference of the first occluding portion 11 to warp to form a flared configuration. The warped first occluding portion 11 cannot provide a clamping force on the rupture, which affects the occlusion performance on the rupture. The positioning disc 116 radially provided adjacent to the distal end of the connecting bolt 112 provides a blocking performance when the connecting bolt 112 pulls the first occluding portion 11 to approach the rupture, ensuring that the first occluding portion 11 is positioned in a false lumen without being pulled into the rupture by the connecting bolt 112, and preventing the edge of the first occluding portion 11 from warping. This ensures that the first occluding portion 11 and the second occluding portion 15 provides a good clamping performance on the rupture. The outer sleeve 111 is engaged with the post 117, the distal end of the metal wire is positioned between the positioning disc 116 and the outer sleeve 111, and the inner sleeve 151 and the outer sleeve 111 may be steel sleeves.

In other embodiments, the occluder 10 may not be provided with the inner sleeve 151 and the outer sleeve 111. The distal ends of the metal wires are converged and positioned at the distal end of the connecting bolt 112, and fixed there by welding. The proximal ends of the metal wires are converged at the distal end of the protrusion portion 152 and positioned by welding

In this embodiment, the inner sleeve 151 is a cylinder with two open ends, and the protrusion portion 152 is also a cylinder with two open ends. The inner diameter of the inner sleeve 151 is larger than the outer diameter of the protrusion portion 152. The inner sleeve 151 may be arranged inside or outside of the second occluding portion 15, and the inner sleeve 151 is mounted around the protrusion portion 152. The proximal ends of the metal wires are located between an inner wall of the inner sleeve 151 and the side wall of the protrusion portion 152. Preferably, the inner sleeve 15 is located inside the second occluding portion 15, the distal end of the protrusion portion 152 extends to the interior of the second occluding portion 15, the inner sleeve 151 is mounted around the distal end of the protrusion portion 152, and the proximal ends of the metal wires bend inwardly and then positioned between the inner wall of the inner sleeve 151 and the side wall of the protrusion portion 152 at the distal end. The inner sleeve 151 is arranged inside the second occluding portion 15. After the occluder 10 is fastened, the length of the second converged member 150 protruding axially beyond the second occluding portion 15 can be reduced, thereby reducing the risk of postoperative complications.

The proximal end of the connecting bolt 112 extends axially towards the proximal end. Before the connecting bolt 112 is screwed in the nut 689, the connecting bolt 112 is located inside the occluder 10, and the proximal end of the connecting bolt 112 extends through the waist 18 of the occluder 10. In this embodiment, the first external thread 113 is provided on the side wall of the connecting bolt 112 at the proximal end thereof, and the second external thread 154 is provided on the side wall of the protrusion portion 152 at the proximal end thereof. In case that the first external thread 113 is a left-hand thread, the second external thread 154 is a right-hand thread; and in case that the first external thread 113 is a right-hand thread, the second external thread 154 is a left-hand thread.

Referring to FIGs. 4 to 7, the pushing component 68 is a steel cable assembly, in which the pushing member 682 is a steel cable with good elasticity, and the pushing tube 684 is a steel tube with good elasticity. The steel cable is movably inserted in the steel duct. The pushing tube 684 includes a body section 6841 and an extension section 6843 disposed at a distal end of the body section 6841. The nut 689 can be engaged in or removed from the extension section 6843. The second internal thread 6842 is located on an inner wall of the extension section 6843 at a distal end thereof. The second internal thread 6842 is engageable with the second external thread 154 of the protrusion portion 152. When the occluder 10 is being connected with the pushing component 68, the screwing direction between the extension section 6843 and the protrusion portion 154 is opposite to the screwing direction between the connecting bolt 112 and the nut 689. The threads of the extension section 6843 and the protrusion portion 154 have a number of turns that is equal to or greater than that of the thread of the nut 689, and the thread pitch in the extension section 6843 and the protrusion portion 154 is equal to or greater than the thread pitch of the nut 689, so that the proximal end of the connecting bolt 112 extends out of a proximal end surface of the nut 689.

The side wall of the pushing member 682 at the distal end thereof is provided with a third external thread 6822 that corresponding to the screw hole 115 of the connecting bolt 112, that is, the first internal thread on the inner wall of the screw hole 115 and the third external thread 6822 are threadedly connected. The distal end of the pushing member 682 can movably extend through the nut 689 arranged in the extension section 6843 and through the extension section 6843. Specifically, A distal end surface of the pushing member 682 is provided with a cylindrical connecting rod 6821 at a central part thereof which protrudes axially therefrom. The outer diameter of the connecting rod 6821 is smaller than the outer diameter of a proximal portion of the pushing member 682. The third external thread 6822 is provided on a side wall at a distal end of the connecting rod 6821.

In the pushing tube 684, the outer diameter of the body section 6841 is smaller than the outer diameter of the extension section 6843, the inner diameter of the body section 6841 is smaller than the inner diameter of the extension section 6843, and thus the nut 689 can abut against a distal port 6845 of the body section 6841. An inner wall at a proximal end of the extension section 6843 is provided with an engaging groove 6846. The nut 689 can be engaged in or released from the engaging groove 6846. The engaging groove 6846 can prevent the nut 689 from rotating in the pushing tube 684. The nut 689 can axially slide in the extension section 6843. Specifically, the engaging groove 6846 is formed by being recessed radially inwardly and regularly from the inner wall of the extension section 6843 at the proximal end. The engaging groove 6846 can be engaged with the nut 689. The nut 689 can be, for example, a hexagonal nut. The engaging groove 6846 is a hexagonal groove corresponding to the hexagonal nut. That is, the radial cross section of the engaging groove 6846 is hexagonal. Therefore, when the nut 689 is engaged in the engaging groove 6846, the nut 689 cannot rotate relative to the pushing tube 684, but the nut 689 axially slides in the extension section 684, and the nut 689 abuts against the distal port 6845.

Referring to Fig. 1, the expander 30 has an elongated cylindrical structure. The diameter of the expander 30 at the distal end thereof is gradually reduced to form a cone-like structure, and a passage (not shown) may be provided therein for a thin filament to extend through. Specifically, the expander 30 includes an expansion rod 32 and a connecting portion 35 provided at a proximal end of the expansion rod 32. A distal end of the expansion rod 32 gradually decreases in diameter to form a cone-like structure. The cone-like structure facilitates the insertion of the expansion rod 32 into the sheath 23. During the operation, it needs to install the expander 30 into the sheath bendable adjustment device 20 to form an expansion assembly.

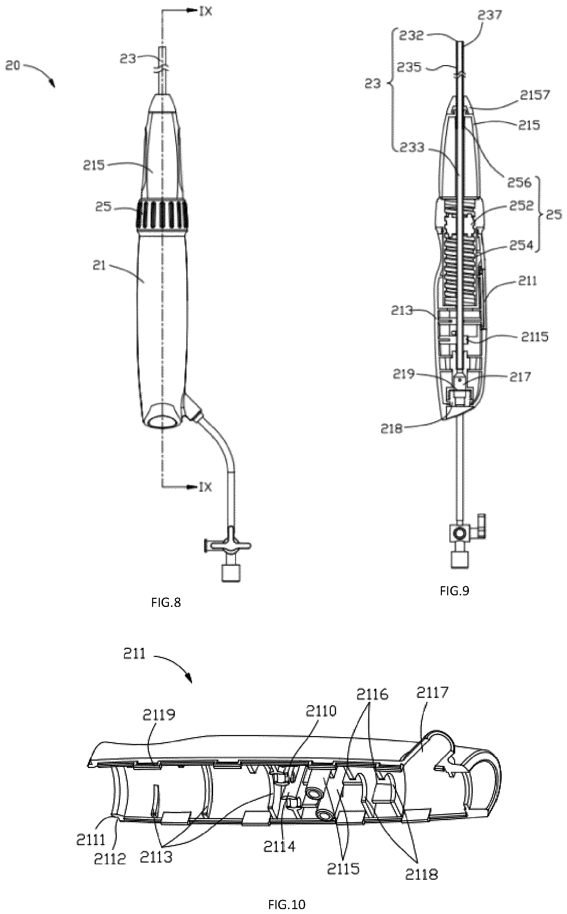

Referring also to FIGs. 8 and 9, the sheath 23 is provided with a bendable distal end, and a proximal end of the sheath 23 is accommodated in the casing 21. Specifically, the sheath 23 includes the bendable distal end and a body section 233. The bendable distal end includes a fixed section 232 located at the distal end and a flexible section 235 connected between the fixed section 232 and the body section 233. A proximal end of the body section 233 is accommodated in the casing 21. The adjustment means 25 includes an adjustment member 252 arranged in the casing 21, a driving member 254 for driving the adjustment member 252 to move along the axial direction of the sheath 23, and two pull wires 256, which are slidably arranged along the axial direction of the sheath 23 at different positions in the peripheral wall of the sheath 23. A wire winding portion 2115 is arranged in the casing 21. Preferably, the wire winding portion 2115 is adjacent to a proximal end of the adjustment member 252. Distal ends of the two pull wires 256 are connected to the bendable distal end. A proximal end of one pull wire 256 is connected to the adjustment member 252, and a proximal end of the other pull wire 256 is wound around the wire winding portion 2115 and is then connected to the adjustment member 252. Specifically, the distal end of one pull wire 256 is fixed to the fixed section 232 of the sheath 23 and the proximal end of the pull wire 256 is directly connected to the adjustment member 252. The distal end of the other pull wire 256 is fixed to the fixed section 232 of the sheath 23, and the proximal end of the other pull wire 256 is wound around the wire winding portion 2115 and is then connected to the adjustment member 252. The driving member 254 can drive the adjustment member 252 to move, which in turn drives the two pull wires 256 to slide, so that the bendable distal end of the sheath 23 bends in different directions. That is, the flexible section 235 of the sheath 23 can bend in different directions.

The sheath bendable adjustment device 20 includes the sheath 23 and the adjustment means 25. The sheath 23 is provided with the bendable distal end, and the proximal end of the sheath 23 is accommodated in the casing 21. The adjustment means 25 includes the adjustment member 252, the driving member 254, and the two pull wires 256, which are slidably provided in the peripheral wall of the sheath 23 at different positions. The distal ends of the two pull wires 256 are respectively connected to the bendable distal end. The proximal end of one pull wire 256 is directly connected to the adjustment member 252, and the proximal end of the other pull wire 256 is wound around the wire winding portion 2101 and is then connected to the adjustment member 252. When the driving member 254 drives the adjustment member 252 to move distally or proximally along the axial direction of the body section 233, the two pull wires 256 can be driven to slide, to thereby bend the bendable distal end of the sheath 23 in different directions. Since the sheath 23 of the sheath bendable adjustment device 20 can be bent in different directions, it can reduce the times of shifting the sheath 23 and the shifting degree of the sheath 23. In addition, the sheath bendable adjustment device 20 is convenient to use, and simple to operate, thereby increasing the work efficiency of sheath bendable adjustment device 20 and improving the success rate of surgery.

The casing 21 includes a first shell 211, a second shell 213, a distal end cap 215, a positioning tube 217 disposed at a proximal end of the casing 21, and a proximal end cap 218 connected to a proximal end of the positioning tube 217. The first shell 211 is connected to the second shell 213 to form a tubular structure with two open ends. The distal end cap 215 is arranged at a distal end of the tubular structure, and the positioning tube 217 is arranged at a proximal end of the tubular structure. The body section 233 of the sheath 23 extends through the distal end cap 215 and the tubular structure and is fixedly connected to the positioning tube 217.

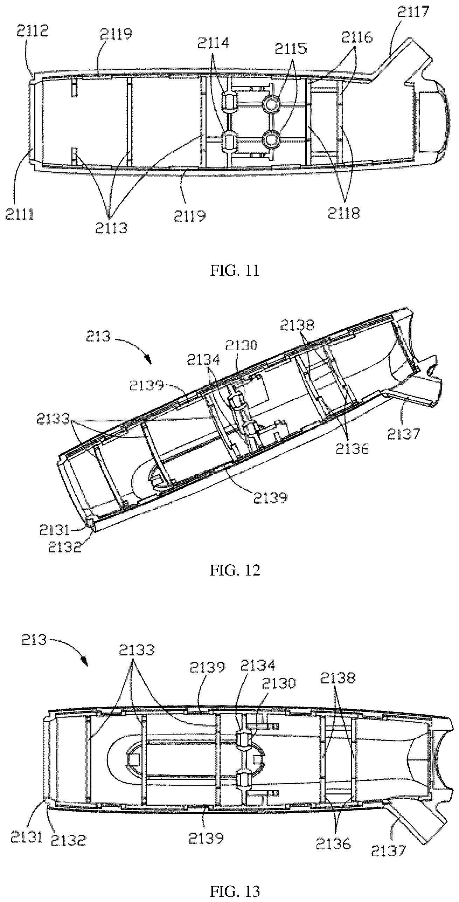

As shown in FIGs. 10 and 11, a distal end surface of the first shell 211 is provided with a semicircular arc-shaped limiting strip 2111 which protrudes axially, and the limiting strip 2111 and the distal end surface of the first shell 211 cooperatively define a semicircular limiting groove 2112. A plurality of first support sheets 2113, two first positioning posts 2114, two wire winding portions 2115, two first positioning sheets 2116, and a semicircular tubular first extension tube 2117 are sequentially arranged on the inner wall of the first shell 211 from the distal end to the proximal end. The plurality of first support sheets 2113 is arranged along the axial direction of the first shell 211 at intervals, and is configured to support the driving member 254. The two first positioning posts 2114 are arranged along the radial direction of the first shell 211 at an interval, and each of the first positioning posts 2114 is provided with a first positioning groove 2110 at the end. The two wire winding portions 2115 are wire winding posts extending along the radial direction the first shell 211, which are arranged along the radial direction of the first shell 211 at an interval. The two first positioning sheets 2116 are arranged along the axial direction of the first shell 211 at an interval. The two first positioning sheets 2116 are each provided with a positioning hole 2118 in the middle thereof and the two positioning holes are corresponding to each other along the axial direction of the first shell 211. The first extension tube 2117 extends obliquely through a side wall of the first shell 211. The two first positioning sheets 2116 and the first extension tube 2117 are used in combination for mounting the positioning tube 217. Two opposite side walls of the first shell 211 are provided with a plurality of engaging pieces 2119 protruding therefrom respectively.

As shown in FIGs. 12 and 13, the structure of the second shell 213 is similar to the structure of the first shell 211. A distal end surface of the second shell 213 is provided with a semi-circular arc-shaped limiting strip 2131 protruding axially therefrom, and the limiting strip 2131 and the distal end surface of the second shell 213 cooperatively define a semicircular limiting groove 2132. A plurality of second support sheets 2133, two second positioning posts 2134, two second positioning sheets 2136, and a semicircular tubular second extension tube 2137 are sequentially arranged on the inner wall of the second shell 213 from the distal end to the proximal end. The plurality of second support sheets 2133 is arranged along the axial direction of the second shell 213 at intervals, and configured to support the driving member 254. The two second positioning posts 2134 are arranged along the radial direction of the second shell 213 at an interval, and each of the second positioning posts 2134 is provided with a second positioning groove 2130 at the end. The two second positioning sheets 2136 are arranged along the axial direction of the second shell 213 at an interval. The two second positioning sheets 2136 are each provided with a positioning hole 2138 in the middle thereof, and the two positioning holes 2138 are corresponding to each other along the axial direction of the second shell 213. The second extension tube 2137 extends obliquely through a side wall of the second shell 213, and the two second positioning sheets 2136 and the second extension tube 2137 are used in combination for receiving the positioning tube 217. Two opposite side walls of the second shell 213 are provided with a plurality of elastic engaging hooks 2139 respectively, and these engaging hooks 2139 are corresponding to with the engaging pieces 2119 of the first shell 211 in a one-to-one manner.

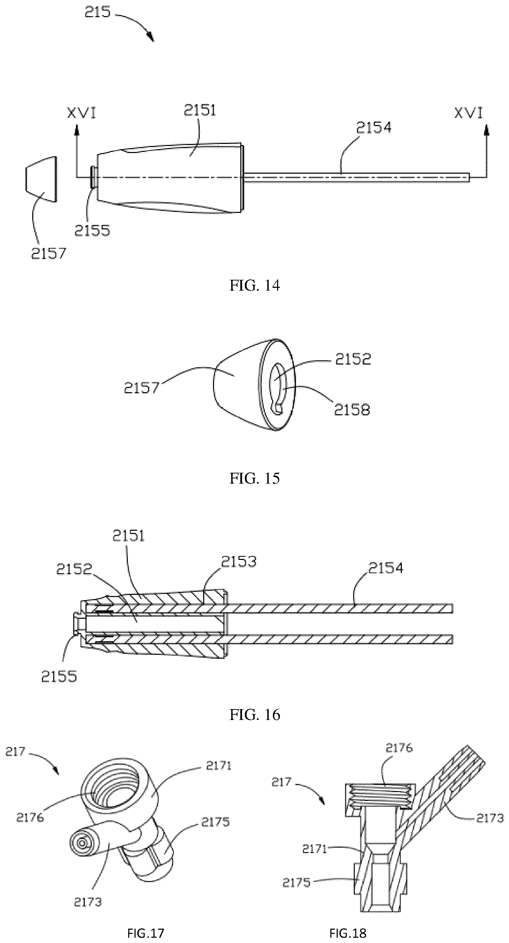

As shown in FIGs. 14 to 16, the distal end cap 215 includes a cap body 2151 and a slipcover 2157 connected to a distal end of the cap body 2151. The distal end cap 215 is axially provided with a through hole 2152, which extends through a central part of the cap body 2151 and the slipcover 2157. The cap body 2151 is provided therein with fixing holes 2153 on opposite sides of the through hole 2152. The fixing holes 2153 extend along the axial direction of the through hole 2152, and the fixing holes 2153 penetrates through a proximal end surface of the cap body 2151. The two fixing holes 2153 are each provided with a guide post 2154 fixed therein. The two guide posts 2154 are spaced apart and parallel with each other, and proximal ends of the guide posts 2154 extend out of the proximal end surface of the distal end cap 215.

In this embodiment, the slipcover 2157 is engaged to the distal end of the cap body 2151. Specifically, an engaging ring 2155 surrounding the through hole 2152 protrudes from the distal end surface of the cap body 2151, and an engaging slot 2158 surround the through hole 2152 is provided at a proximal end of the slipcover 2157 corresponding to the engaging ring 2155. The engaging ring 2155 can be engaged in the engaging slot 2158. The slipcover 2157 is made of a soft material such as rubber, and silicone, etc. The proximal end of the sheath 23 extends through the through hole 2152 of the slipcover 2157 and the cap body 2151 and is then connected between the first shell 211 and the second shell 213. Because the slipcover 2157 is made of a soft material, the slipcover 2157 can reduce the friction and wear between the sheath 23 and the distal end port of the distal end cap due to wobbling.

As shown in FIGs. 17 and 18, the positioning tube 217 is a Y-shaped tube. The positioning tube 217 includes a main tube 2171 and a side tube 2173 obliquely protruding from a proximal end of the main tube 2171. The side tube 2173 communicates with the main tube 2171. A ring-shaped bulge 2175 protrudes from an outer peripheral surface of the main tube 2171 at a distal end. The axial extension length of the bulge 2175 is equal to a distance between the two first positioning sheets 2116 of the first shell 211. A proximal end of the main tube 2171 is provided with an internal thread 2176 and the internal thread 2176 is used to connect the proximal end cap 218. A distal end of the positioning tube 217 is configured to connect with and fix the body section 233 of the sheath 23.

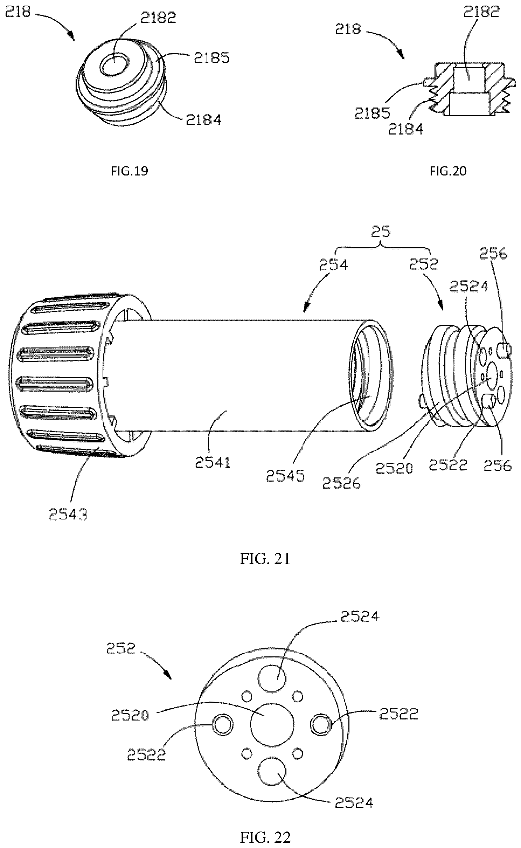

As shown in FIGs. 19 and 20, the proximal end cap 218 is cylindrical, and a central portion of the proximal end cap 218 is axially provided with a through hole 2182. A side wall of the proximal end cap 218 at a distal end is provided with an external thread 2184 corresponding to the internal thread 2176 of the positioning tube 217. A ring-shaped stop piece 2185 protrudes from a side wall of the proximal end cap 218 at a proximal end thereof. The stop piece 2185 can increase the tightness of threaded connection between the proximal end cap 218 and the positioning tube 217.

As shown in FIGs. 21 and 22, the adjustment member 252 is threadedly connected to the driving member 254. Rotation of the driving member 254 can drive the adjustment member 252 to move along the axial direction of the sheath 23. Specifically, the adjustment member 252 is cylindrical, with a through hole 2520 provided in a central portion thereof along the axial direction. The adjustment member 252 is slidably mounted around the sheath 23 by means of the through hole 2520. The adjustment member 252 is provided with two fixing holes 2522 on two opposite sides of the through hole 2520, and the two fixing holes 2522 are configured to fix the proximal ends of the two pull wires 256 respectively. The adjustment member 252 is provided with two guide sliding holes 2524 on another two opposite sides of the through hole 2520. The two guide holes 2524 correspond to the two guide posts 2154 respectively. That is, the two guide posts 2154 can be slidably inserted into the two guide holes 2524. The outer periphery of the adjustment member 252 is provided with an external thread 2526.

In this embodiment, the axes of the two fixing holes 2522 are symmetrical with respect to the axis of the through hole 2520, and the axes of the two fixing holes 2522 are coplanar with the axis of the through hole 2520. The axes of the two guide holes 2524 are symmetrical with respect to the axis of the through hole 2520, and the axes of the two guide holes 2524 are coplanar with the axis of the through hole 2520. The plane defined by the axes of the two fixing holes 2522 is perpendicular to the plane defined by the axes of the two guide holes 2524.

The driving member 254 includes a rotatable cylinder 2541 and an operating portion 2543 provided on the rotatable cylinder 2541. Specifically, one end of the driving member 254 is provided with the rotatable cylinder 2541, and the operating portion 2543 is provided on a distal side wall of the rotatable cylinder 2541. An inner peripheral surface of the rotatable cylinder 2541 is provided with an internal thread 2545 corresponding to the external thread 2526 of the adjustment member 252. The adjustment member 252 can be received in the rotatable cylinder 2541, and the outer peripheral surface of the adjustment member 252 is threadedly connected to the inner peripheral surface of the rotatable cylinder 254. That is, the external thread 2526 is screwed to the internal thread 2545. The operating portion 2543 is an annular sleeve, which is fixedly mounted around the rotatable cylinder 2541, and rotation of the operating portion 2543 can drive the rotating cylinder 2541 to rotate together. An outer peripheral surface of the operating portion 2543 is provided with anti-slip strips 2546, and these anti-slip strips 2546 facilitate the operation of the driving member 254.

In other embodiments, the rotatable cylinder 2541 and the operating portion 2543 can be made integrally.

In other embodiments, the rotatable cylinder 2541 and the operating portion 2543 can be fixed by screwing, gluing, welding etc.

In other embodiments, the outer peripheral surface of the operating portion 2543 may be provided with anti-slip textures or roughening treatments.

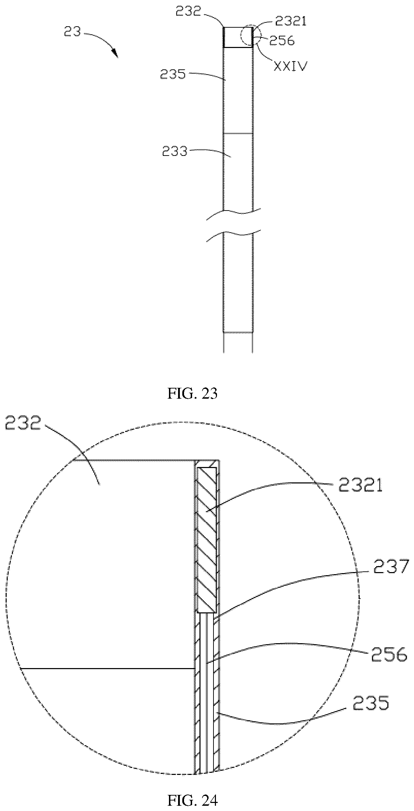

As shown in FIGs. 23 and 24, the two pull wires 256 are located at opposite ends of the sheath 23 in the diametrical direction and the distal ends of the pull wires 256 are both fixed to the fixed section 232. The pull wire 256 can slide axially in the peripheral wall of the sheath 23. The flexible section 235 is made of a flexible material, and the flexible section 235 can be elastically restored to its original shape after being bent for 180 degrees. The fixed section 232 of the sheath 23 is provided therein with an annular imaging ring 2321, and the distal ends of the two pull wires 256 are respectively fixed to the proximal end of the imaging ring 2321. Preferably, two pull tubes 237 are arranged in the peripheral wall of the sheath 23 which are opposite to each other, and each pull tube 237 extends along the axial direction of the sheath 23. The two pull tubes 237 are located at two ends in the radial direction of the sheath 23. The distal end of each pull tube 237 is connected to the fixed section 232 of the sheath 23. Specifically, the pull tube 237 is fixed to the peripheral wall at the proximal end of the imaging ring 2321 by welding or bonding. The proximal end of each pull tube 237 extends along the peripheral wall of the sheath 23 into the casing 21, and the proximal end port of each pull tube 237 bends away from the axis and extends into the casing 21. The two pull wires 256 are slidably inserted into the two pull tubes 237 respectively. The distal end of each pull wire 256 is fixed to the imaging ring 2321 in the fixed section 232 by welding. The proximal end of the pull wire 256 extends out of the proximal end port of the pull tube 237 and is then connected to the adjustment member 252. The pull wire 256 can slide along the corresponding pull tube 237.

In this embodiment, the two pull tubes 237 are located at the two ends in the diametrical direction of the sheath 23, and the axes of the two pull tubes 237 are in the same plane with the axis of the sheath 23.

The outer peripheral surface of the flexible section 235 of the sheath 23 is recessed to form two bending facilitating grooves 2351 at positions corresponding to the two pull wires 256. Specifically, the two bending facilitating grooves 2351 are corresponding to the two pull tubes 237 in a in one-to-one manner. Each bending facilitating groove 2351 extends along the axial direction of the sheath 23 to the distal end of the body section 233. With the pulling force of the pull wire 256, the sheath 23 causes the stress to concentrate in the corresponding bending facilitating groove 2351, which facilitates the flexible section 235 to bend along the direction of the bending facilitating groove 2351 upon a smaller force of the corresponding pull wire 256, thereby improving the accuracy of the bending direction and the ease of bending of the flexible section 235, and the simplicity of operation.

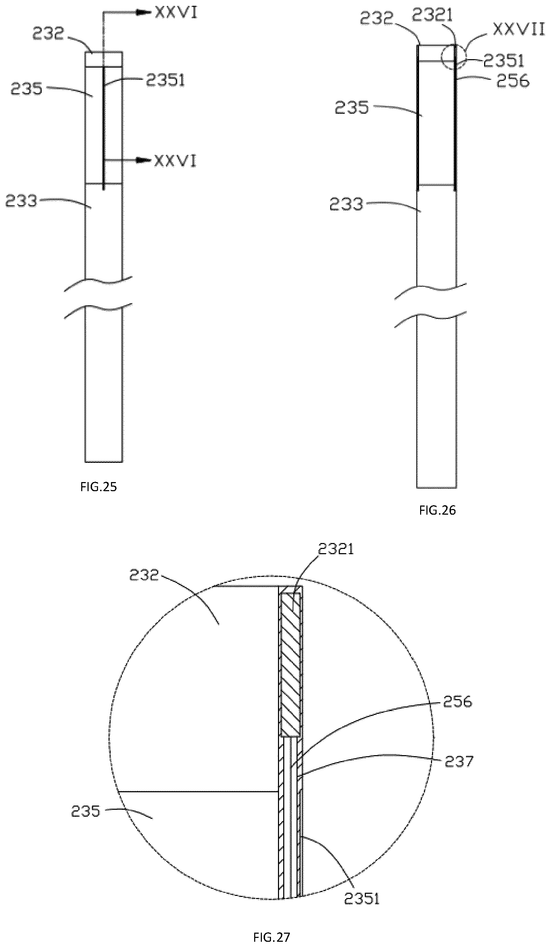

Referring to FIGs. 8 to 28, when the sheath bendable adjustment device 20 is assembled, firstly, the adjustment member 252 is screwed into the rotatable cylinder 2541 of the driving member 254, and a gasket 255 is mounted around the rotatable cylinder 2541 from the proximal end thereof, until the gasket 255 abuts against the proximal end surface of the operating portion 2543, wherein the gasket 255 facilitates the rotation of the driving member 254. The proximal end of the body section 233 of the sheath 23 is caused to slidably extend through the through hole 2520 of the adjustment member 252 from the distal end of the rotatable cylinder 2541, and is clamped at the distal end of the positioning tube 217. The rotatable cylinder 2541 of the driving member 254 is positioned on the first support sheets 2113 of the first shell 211. The bulge 2175 of the positioning tube 217 is engaged between two first positioning sheets 2116, and the portions of the positioning tube 217 at two ends of the bulge 2175 are engaged in the positioning holes 2118 of two first positioning sheets 2116. by this time, the operating portion 2543 of the driving member 254 is exposed from the distal end of the first shell 211, and a part of the gasket 255 is engaged in the limiting groove 2112 of the first shell 211. The sheath 23 extends through the gap between the two first positioning posts 2114 and the gap between the two wire winding portions 2115. The side tube 2173 of the positioning tube 217 is inserted into the first extension tube 2117.

The proximal end of one pull wire 256 is fixed to the distal end of one fixing hole 2522 of the adjustment member 252, and the proximal end of the other pull wire 256 is wound around the wire winding portion 2115 and is then fixed to the distal end of the other fixing hole 2522. by this time, the two pull wires 256 are located at two opposite sides of the axis of the sheath 23, and the two pull wires 256 are in a tensioned state; and the flexible section 235 of the sheath 23 is straight.

The slipcover 2157 is mounted around the distal end of the cap body 2151, and the proximal ends of the two guide sliding posts 2154 of the distal end cap 215 are slidably extended through the two guide sliding holes 2524 of the adjustment member 252 from the distal end of the rotatable cylinder 2541, until the proximal ends of the guide sliding posts 2154 are engaged in the first positioning grooves 2110 of the two first positioning posts 2114 respectively. by this time, the distal end of the sheath 23 is exposed after extending through the through hole 2152 of the cap body 2151 and the slipcover 2157. The second shell 213 is fitted to the first shell 211, such that the other part of the gasket 255 is engaged in the limiting groove 2132 of the second shell 213, and the engaging hooks 2139 of the second shell 213 are engaged with the corresponding engaging piece 2119 of the first shell 211, so that the adjustment means 25 is mounted between the first shell 211 and the second shell 213. by this time, the second support sheets 2133 of the second shell 213 are corresponding to the first support sheets 2113 of the first shell 211 in a one-to-one manner. The opposed first support sheet 2113 and the second support sheet 2132 cooperatively define a circular hole for mounting the rotatable cylinder 2541, and the driving member 254 can rotate in the circular hole. The second positioning posts 2134 of the second shell 213 are corresponding to the first positioning posts 2114 of the first shell 211 in a one-to-one manner, and the first positioning groove 2110 and second positioning groove 2130 of the corresponding first positioning post 2114 and the second positioning post 2134 cooperatively define a space for engaging the proximal end of the sheath 23. The ends of the two wire winding portions 2115 abut against the inner wall of the second shell 213, which prevents the pull wire 256 from release from the wire winding portions 2115. The second positioning sheets 2136 of the second shell 213 are corresponding to the first positioning sheets 2116 of the first shell 211 in a one-to-one manner, and the positioning hole 2118 and the positioning hole 2138 of the corresponding first positioning sheet 2116 and the second positioning sheet 2136 cooperatively define a space for mounting the main tube 2171 of the positioning tube 217. Then the external thread 2184 of the proximal end cap 218 is screwed to the internal thread 2176 at the proximal end of the main tube 2171, and a seal ring 219 is provided between the proximal end cap 218 and the main tube 2171. The seal ring 219 is configured to prevent the blood or other liquid in the sheath 23 from flowing out from the proximal end of the proximal end cap 21 during an interventional operation.

The rotational movement of the operating portion 2543 of the driving member 254 in the circumferential direction is transformed into the back-and-forth movement of the adjustment member 252 in the axial direction. Specifically, the operating portion 2543 is sandwiched between the first shell 211 and the second shell 213, and the distal end cap 215, and is fixed in the axial direction relative to the casing 21. That is, the operating portion 2543 cannot move back and forth in the axial direction, while the operating portion 2543 can rotate in the circumferential direction. The two guide posts 2154 are fixed in the distal end cap 215, and thus fixed relative to the casing 21, as the two guide posts 2154 are extended into the two guide holes 2524 of the adjustment member 252, the movement of the adjustment member 252 is limited to back-and-forth movement relative to the axial direction of the casing 21, and the adjustment member 252 is fixed in the circumferential direction. When the operating portion 2543 is rotated, the adjustment member 252 received in the driving member 254 cannot rotate in the circumferential direction, and relative rotation occurs between the external thread 2526 of the adjustment member 252 and the internal thread 2545 of the driving member 254, so that the relative positions of the adjustment member 252 and the driving member 254 in the axial direction change, causing the adjustment member 252 to move back and forth in the axial direction.

That is, when the sheath bendable adjustment device 20 is used, the driving member 254 is rotated about the axis by operating the operating portion 2543, to drive the adjustment member 252 to move proximally or distally along the guide posts 2154. In an initial state of the adjustment means 25, when the adjustment member 252 is moved proximally, the pull wire 256 directly fixed to the adjustment member 252 can be driven to slide proximally, so that the flexible section 235 is bent towards the side of this pull wire 256. During the bending process of the flexible section 235, the pull wire 256 which is wound around the wire winding portion 2115 and then connected to the adjustment member 252 is pulled to slide distally by the deformation of the flexible section 235, to allow the flexible section 235 to achieve the bending. In the initial state of the adjustment means 25, when the adjustment member 252 is moved distally, the pull wire 256 which is wound around the wire winding portion 2115 and then connected to the adjustment member 252 is driven by the adjustment member 252 to slide proximally, so that the flexible section 235 is bent towards the side of this pull wire 256. During the bending process of the flexible section 235, the pull wire 256 directly fixed to the adjustment member 252 is driven to slide distally, to allow the flexible section 235 to achieve the bending.

The initial state of the adjustment means 25 refers to a state that the flexible section 235 of the sheath 23 is straight.

Since the adjustment member 252 and the driving member 254 are threadedly connected, when there is a need to bend the flexible section 235 of the sheath 23, it only requires the operating portion 2543 be operated to rotate the driving member 254 to drive the adjustment member 252 to move forth or back along the axial direction of the sheath 23, thereby driving the flexible section 235 of the sheath 23 to bend to a proper position. When the rotatable cylinder 2541 stops to rotate, the adjustment member 252 is positioned. Therefore, the flexible section 235 of the sheath 23 of the sheath bendable adjustment device 20 in the present application can be bent through any angle and positioned at any angle. The sheath bendable adjustment device 20 is simple to operate and convenient to use, and can improve the work efficiency and the success rate of surgery.

In other embodiments, the rotatable cylinder 2541 of the driving member 254 of the adjustment means 25 can be replaced by a screw bolt, and the adjustment member 252 is axially provided with a threaded through hole corresponding to the screw. The screw bolt is screwed into the threaded through hole, and the adjustment member 252 can be driven to reciprocate axially by rotating the screw bolt. Specifically, the screw bolt is arranged in the central part of the radial cross section of the operating portion 2543 and extends axially. A through hole is axially provided in a central portion of the screw bolt. The threaded through hole is provided in a central part of the radial cross section of adjustment member 252 and axially extends through the proximal end surface and distal end surface of the adjustment member 252. After the screw bolt is screwed to the threaded through hole, the proximal end of the sheath 23 can slidably extend through the through hole of the screw bolt and the threaded through hole. The guide post 2154 can also slidably extend through the through hole of the screw bolt

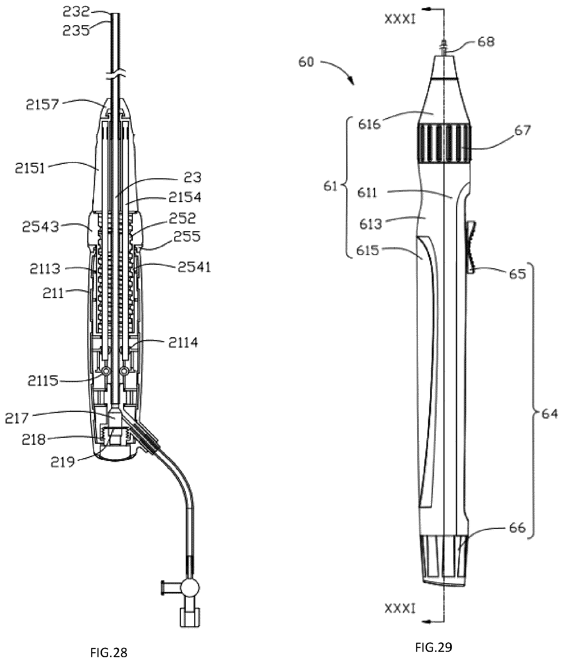

Referring also to FIGs. 29 to 31, the first control component 64 includes a sliding means 65 and a first rotating means 66. The sliding means 65 can drive the pushing member 682 to slide axially and be positioned in the pushing tube 684, that is, the sliding means 65 can drive the pushing member 682 to slide axially and be positioned relative to the pushing tube. The first rotating means 66 can drive the pushing member 682 to rotate in the pushing tube 684, that is, the first rotating means 66 can drive the pushing member 682 to rotate relative to the pushing tube 684. The second control component 67 includes a second rotating means 670. The second rotating means 670 can drive the pushing tube 684 to rotate outside the pushing member 682, that is, the second rotating means 670 can drive the pushing tube 684 to rotate with respect to the pushing member 682.

The sliding means 65 of the pushing device 60 can drive the pushing member 682 to slide axially in the pushing tube 684, which in turn drives the proximal end of the connecting bolt 112 to extend through the through hole 153 of the protrusion portion 152 to abut against or move away from the nut 689 positioned in the pushing tube 684. The first rotating means 66 can drive the pushing member 682 to rotate in the pushing tube 684, to realize the third external thread 6822 of the pushing member 682 being threaded connected with or released from the screw hole 115 of the connecting bolt 112. The second rotating means 670 can drive the pushing tube 684 to rotate outside the pushing member 682, to thereby drive the nut 689 to rotate, so that the nut 689 can be screwed to the connecting bolt 112 and the second external thread 154 of the protrusion portion 152 is disengaged from the second internal thread 6842 of the pushing tube 684; or so that the nut 689 is disengaged from the connecting bolt 112 and the second external thread 154 of the protrusion portion 152 is screwed to the second internal thread 6842 of the pushing tube 684. The pushing device 60 is simple to operate and convenient to use, which improves the reliability and efficiency of the pushing device 60, thereby reducing the operation time.

The housing 61 includes a first shell 611, a second shell 613, an outer buckle plate 615 and an end cover 616. The first shell 611 is fitted to the second shell 613 to form a tubular structure with two open ends. The sliding means 65 is axially arranged in the tubular structure. The first rotating means 66 is arranged at a proximal end of the tubular structure. The second rotating means 670 is arranged at a distal end of the tubular structure. The end cover 616 is arranged at a distal end of the second rotating means 670.

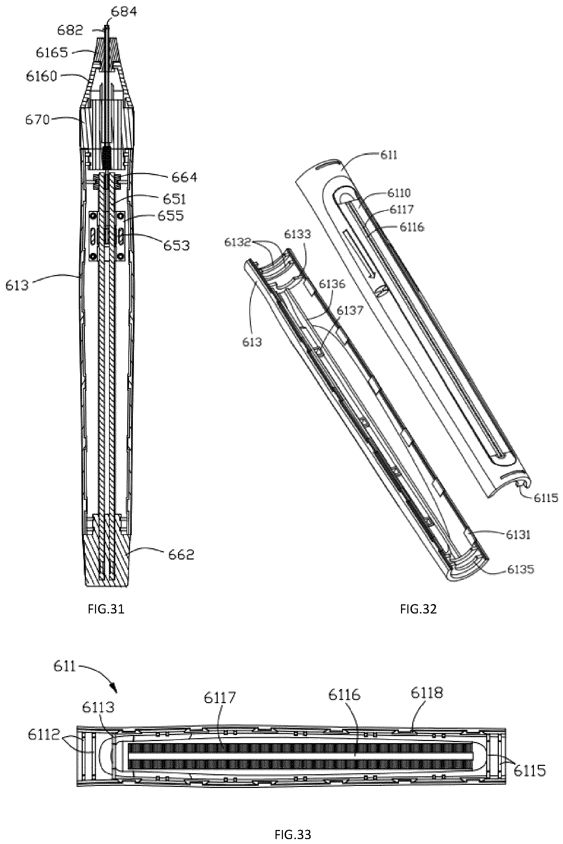

As shown in FIGs. 32 and 33, an inner wall of the first shell 611 is provided with at least one first support sheet 6112 and at least one second support sheet 6113 at a distal end thereof along the axial direction. The inner wall of the first shell 611 is provided with at least one third support sheet 6115 at a proximal end thereof. The first support sheet 6112, the second support sheet 6113 and the third support sheet 6115 are all semi-circular ring-shaped sheets. The first support sheet 6112 and the second support sheet 6113 are arranged at an interval. The axes of the first support sheet 6112, the second support sheet 6113 and the third support sheet 6115 all extend along the axial direction, and the axes of the first support sheet 6112, the second support sheet 6113 and the third support sheet 6115 coincide each other.

In this embodiment, two first support sheets 6112 are provided, and the two first support sheets 6112 are spaced apart axially and parallel with each other. One second support sheet 6113 is provided. Two third support sheets 6115 are provided, and the two third support sheets 6115 are spaced apart axially and parallel with each other.

In other embodiments, other numbers of the first support sheet 6112, the second support sheet 6113, and the third support sheet 6115 may be provided.

The side wall of the first shell 611 is configured to have a rectangular plane 6110, and the length of the plane 6110 extends along the axial direction, that is, the plane 6110 extends from the distal end to the proximal end. The plane 6110 is provided with a strip-shaped opening 6116 along the axial direction. The strip-shaped opening 6116 is located between the second support sheet 6113 and the third support sheet 6115. The strip-shaped opening 6116 extends from the distal end to the proximal end of the first shell 611. The inner wall of the first shell 611 is provided with a rack 6117 on at least one side of the strip-shaped opening 6116, and the rack 6117 extends from the distal end to the proximal end of the strip-shaped opening 6116. Two opposite side walls of the first shell 611 are respectively provided with a plurality of engaging pieces 6118 protruding there from.

In this embodiment, the inner wall of the first shell 611 is provided with two strip-shaped grooves along the axial direction on opposite sides of the strip-shaped opening 6116, and each strip-shaped groove is provided with a rack 6117.

As shown in FIGs. 32, 34 and 35, the structure of the second shell 613 is similar to the structure of the first shell 611. An inner wall of the second shell 613 is provided with at least one fourth support sheet 6132 and at least one fifth support sheet 6133 at a distal end thereof along the axial direction. The inner wall of the second shell 613 is provided with at least one sixth support sheet 6135 at a proximal end thereof. The fourth support sheet 6132, the fifth support sheet 6133 and the sixth support sheet 6135 are all semi-circular ring-shaped sheets. The fourth support sheet 6132 and the fifth support sheet 6133 are arranged at an interval. The axes of the fourth support sheet 6132, the fifth support sheet 6133 and the sixth support sheet 6135 all extend along the axial direction, and the axes of the fourth support sheet 6132, the fifth support sheet 6133 and the sixth support sheet 6135 coincide. When the first shell 611 is fitted to the second shell 613, the first support sheet 6112, the second support sheet 6113 and the third support sheet 6115 of the first shell 611 are respectively corresponding to the fourth support sheet 6132, the fifth support sheet 6133 and the sixth support sheet 6135 of the second shell 613. The first support sheet 6112 and the corresponding fourth support sheet 6132 cooperatively define a ring-shaped support sheet, the second support sheet 6113 and the fifth support sheet 6133 cooperatively define a ring-shaped support sheet, and the third support sheet 6115 and the corresponding sixth support sheet 6135 cooperatively define a ring-shaped support sheet.

In this embodiment, two fourth support sheets 6132 are provided, and the two fourth support sheets 6132 are spaced apart and parallel with each other. One fifth support sheet 6133 is provided. Two sixth support sheets 6135 are provided, and the two sixth support sheets 6135 are spaced apart and parallel with each other.

In other embodiments, other numbers of the fourth support sheet 6132, the fifth support sheet 6133, and the sixth support sheet 6135 may be provided.

At least one rail 6136 protrudes from the inner wall of the second shell 613 along the axial direction. The rail 6136 is located between the fifth support sheet 6133 and the sixth support sheet rail 6135, and the rail 6136 extends from the distal end to the proximal end. In this embodiment, two rails 6136 spaced apart and parallel with each other protrude from the middle portion of the inner wall of the second shell 613, and a plurality of positioning blocks 6137 are axially arranged at intervals between the two rails 6136. Each positioning block 6137 is provided with an engaging hole. The side wall of the second shell 613 is provided with a mounting opening 6138 at a central part thereof, and the second shell 613 is provided with a plurality of engaging holes 6139 around the mounting opening 6138. Two opposite side walls of the second shell 613 are respectively provided with a plurality of elastic engaging hooks 6131 protruding therefrom, which are corresponding to the engaging pieces 6118 of the first shell 611 in a one-to-one manner.

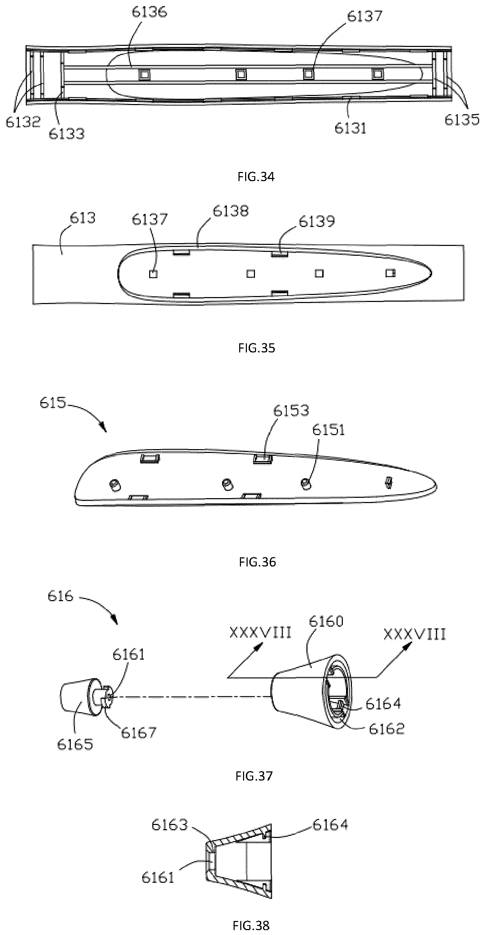

As shown in FIG. 36, the outer buckle plate 615 is a convex arc-shaped sheet. The outer buckle plate 615 is corresponding to the mounting opening 6138 of the second shell 613. A side of the outer buckle plate 615 facing the mounting opening 6138 is provided with a plurality of positioning posts 6151 and a plurality of engaging hooks 6153 protruding therefrom. These positioning posts 6151 are corresponding to the engaging holes in the positioning blocks 6137 of the second shell 613 in a one-to-one manner. These engaging hooks 6153 are corresponding to the engaging holes 6139 of the second shell 613 in a one-to-one manner. The side wall of the outer buckle plate 615 is provided with anti-slip strips or bulges to increase the friction, for facilitating the grip of the pushing device 60 by fingers and facilitating the operation of the pushing device 60.

As shown in FIGs. 37 and 38, the end cover 616 has a conical structure. The end cover 616 includes a cover body 6160 and a slipcover 6165 connected to a distal end of the cover body 6160. The end cover 616 is axially provided with a through hole 6161, and the through hole 6161 extends through a central portion of the cover body 6160 and the slipcover 6165. A tapered cavity is defined inside the cover body 6160, and an annular groove 6162 is provided on an inner wall of the cover body 6160 at the proximal end. The axis of the annular groove 6162 coincides with the axis of the through hole 6161. Two opposing engaging blocks 6164 are arranged in the tapered cavity at the proximal end of the cover body 6160.

The slipcover 6165 is engaged to the distal end of the cover body 6160. Specifically, an engaging ring protrudes from an inner peripheral surface of the through hole 6161 at the distal end of the cover body 6160, and an extension post surrounding the through hole 6161 protrudes from a proximal end surface of the slipcover 6165. A proximal end of the extension post is radially provided with a pair of protruding sheets. After the slipcover 6165 is inserted into the through hole 6161 of the cover body 6160 by means of the extension tube, the protruding sheet 6167 is engaged to the engaging ring 6163 of the cover body 6160, to fix the slipcover 6165 to the cover body 6160. The slipcover 6165 is made of soft materials such as rubber and silicone. The proximal end of the pushing component 68 extends through the through hole 6161 of the slipcover 6165 and the cover body 6160 and is then connected into the housing 61. Since the slipcover 6165 is made of a soft material, the slipcover 6165 can reduce the friction and wear of the pushing component 68 with the distal end port of the end cover 616 caused by the wobbling.

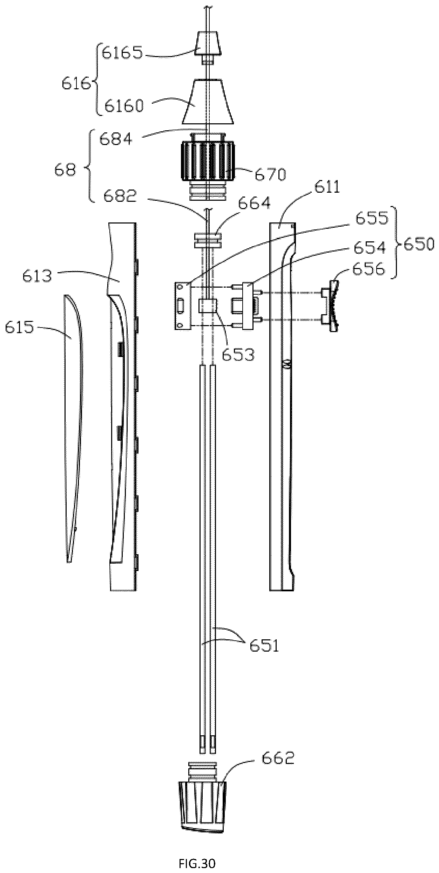

Referring to FIGs 30, 31 and 39 to 43, the sliding means 65 is slidably disposed in the housing 61 along the axial direction. The sliding means 65 includes at least one guide rail 651 that is disposed to extend in the housing 61 along the axial direction, a movable block 653 that is slidably arranged around the at least one guide rail 651, and a sliding member 650 that can drive the movable block 653 to slide along the guide rail 651. A proximal end of the pushing member 682 is fixed to the movable block 653. The sliding member 650 includes a first clamping block 654, a second clamping block 655, and a button 656 partially exposed from the housing 61 and connected to the sliding member 650. The first clamping block 654 can be moved close to or away from the second clamping block 655. The movable block 653 is arranged between the first clamping block 654 and the second clamping block 655. The first clamping block 654 and the second clamping block 655 can be moved close to each other to tightly clamp the movable block 653 therebetween. The first clamping block 654 and the second clamping block 655 can also be moved away from each other to release the movable block 653. The movable block 653 can rotate.

In this embodiment, two guide rails 651 are provided in the housing 61 which are axially arranged, spaced apart and parallel with each other.

The first clamping block 654 has a semi-circular tube structure. A rectangular top surface 6541 is provided in a central part of a side wall of the first clamping block 654. The length direction of the top surface 6541 extends along the axial direction of the first clamping block 654. Partial structure of the first clamping block 654 can extend through the strip-shaped opening 6116 and slide there along. Specifically, a sliding strip 6542 protrudes from a central part of the top surface 6541, and the length direction of the sliding strip 6542 extends along the length direction of the top surface 6541. The sliding strip 6542 can be inserted into the strip-shaped opening 6116 of the first shell 611 and is slidable therein. The top surface 6541 is provided with engaging teeth 6544 on at least one side of the sliding strip 6542. The engaging teeth 6544 can be engaged to the corresponding rack 6117 of the first shell 611. Preferably, the top surface 6541 is provided with engaging teeth 6544 on two opposite sides of the sliding strip 6542, and the engaging teeth 6544 on two opposite sides can be respectively engaged to the two racks 6117 of the first shell 611. Two connecting posts 6545 respectively protrudes from the two opposite ends of the sliding strip 6542 on the top surface 6541. Two sides of the first clamping block 654 facing away from the top surface 6541 are each provided with two guide posts 6546 and a guide plate 6547 protruding therefrom. On each side, the guide plate 6547 is located between the two guide posts 6546. The first clamping block 654 is provided with a first clamping groove 6548 on an inner wall thereof at a central part, and the first clamping groove 6548 can receive the side wall of the movable block 653.

The second clamping block 655 has a semi-circular tube structure. Two chutes 6552 spaced apart from each other are axially provided in a central part of a side wall of the second clamping block 655. The two chutes 6552 correspond to the rails 6136 of the second shell 613, and the second clamping block 655 is slidable along the rails 6136. Two opposite sides of the second clamping block 655 each are provided with two guide holes 6554 and an access hole 6556. On each side, the access hole 6556 is located between the two guide holes 6554. A second clamping groove 6558 is provided in a central part of an inner wall of the second clamping block 655, and the second clamping groove 6558 can receive the side wall of the movable block 653.

The movable block 653 has a cylindrical structure. The proximal end of the pushing member 682 is fixed to a central part of the movable block 653. Specifically, a central part of a distal end surface of the movable block 653 is provided with a fixing hole 6532 along the axial direction, and the proximal end of the pushing member 682 is fixed in the fixing hole 6532. The end surface of the movable block 653 is provided with two through holes 6534 at two opposite sides of the fixing hole 6532. Each through hole 6534 extends in the axial direction and penetrates through the proximal end surface and the distal end surface of the movable block 653. Each through hole 6534 is configured for one guide rail 651 extending therein. The side wall of the movable block 653 is provided with a plurality of welding holes 6535 communicating with the fixing hole 6532, and the pushing member 682 is fixed to the movable block 653 by solder added into the welding hole 6535.

An elastic member 658 is provided between the first clamping block 654 and the second clamping block 655, and the elastic member 658 can force the first clamping block 654 to move away from the second clamping block 655. Preferably, the elastic member 658 is a spring mounted outside the guide post 6546.

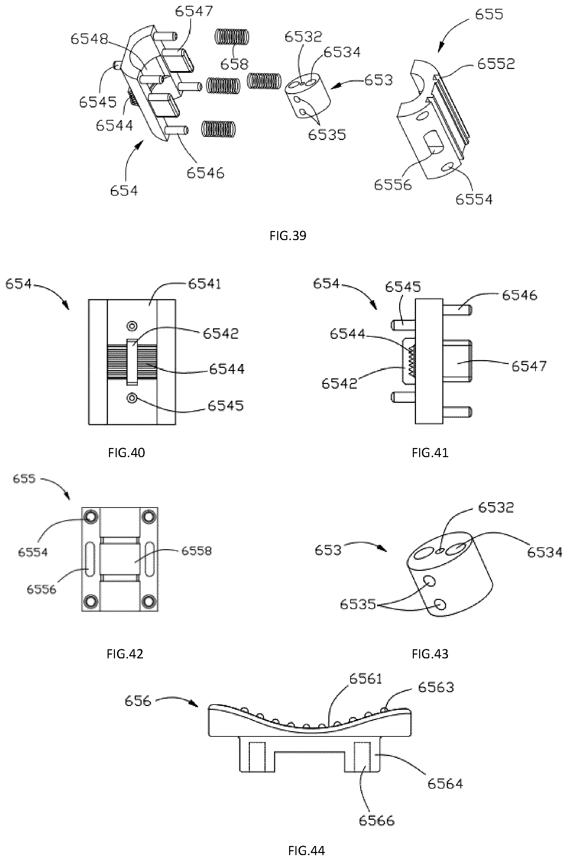

As shown in FIG. 44, a recess 6561 is provided in a central part of the side wall of the button 656, and a number of anti-slip strips 6563 are arranged on the surface of the recess 6561. A finger can be inserted into the recess 6561 to facilitate the operation of the button 656. Two connecting blocks 6564 respectively protrudes from a side of the button 656 facing away from the recess 6561 at two opposite ends. Each connecting block 6564 is provided with a connecting hole 6566 on a side facing away from the recess 6561. The two connecting holes 6566 are corresponding to the two connecting posts 6545 of the first clamping block 654.

In other embodiments, the button 656 may be integrally formed with the first clamping block 654, that is, the first clamping block 654 is provided with a button extending through the strip-shaped opening 6116.

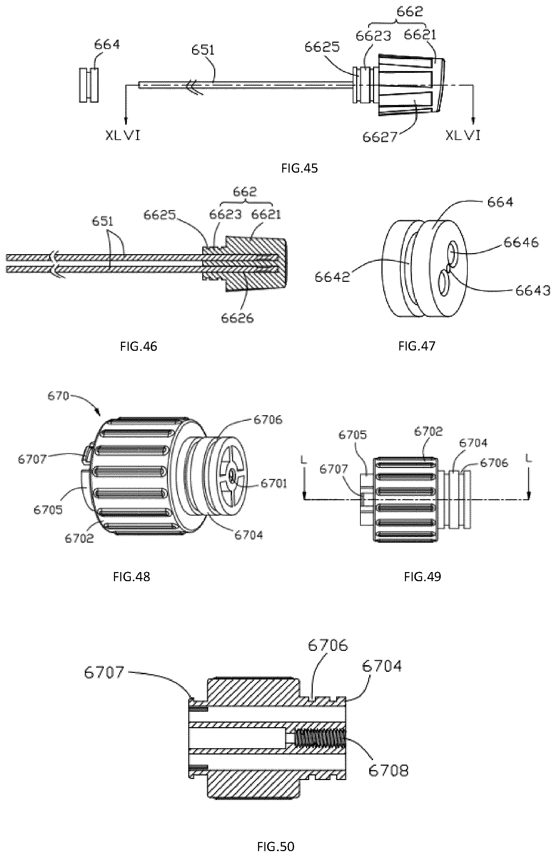

Referring to FIGs. 45 to 47, the first rotating means 66 includes a first rotating member 662 fixed to the guide rail 651, and a rotating post 664 arranged in the housing 61 which is rotatable about the axis of the pushing member 682. Specifically, the first rotating means 66 is fixedly connected to the proximal end of the guide rail 651, and the rotating post 664 is fixedly connected to the distal end of the guide rail 651. The axis of the guide rail 651 is spaced apart from and parallel to the axis of the pushing member 682. The first rotating member 662 is arranged at the proximal end of the housing 61 rotatable about the axis of the pushing member 682. The rotation of the first rotating member 662 can drive the movable block 653 and the rotating column 664 to rotate through the guide rail 651, thereby causing the pushing member 682 to rotate.

Specifically, the first rotating member 662 includes a handle portion 6621 and a rotating rod 6623 protruding from a central part of a distal end surface of the handle portion 6621. The rotating rod 6623 is a cylinder. A side wall of the rotating rod 6623 is provided with two annular guide grooves 6625 along its circumferential direction. The two guide grooves 6625 correspond to the ring-shaped support sheets defined by the third support sheets 6115 of the first shell 611 and the sixth support sheets 6135 of the second shell 613. The axes of the two guide grooves 6625 coincide with the axis of the first rotating member 662. The first rotating member 662 can rotate along the guide groove 6625. An end surface of the rotating rod 6623 facing away from the handle portion 6621 is provided with two spaced fixing holes 6626. The axes of the two fixing holes 6626 do not coincide with the axis of the rotating rod 6623. The proximal ends of the two guide rails 651 are fixed respectively in the two fixing holes 6626. A number of anti-slip grooves 6627 are provided on the side wall of the handle portion 6621 to facilitate the rotation of the first rotating member 662.

The rotating post 664 is a cylinder. A side wall of the rotating column 664 is provided with a guide groove 6642 along its circumference. The guide groove 6642 corresponds to the ring-shaped support sheet defined by the second support sheet 6113 of the first shell 611 and the fifth support sheet 6133 of the second shell 613. The rotating post 664 can rotate along the guide groove 6642. A central part of the rotating post 664 is axially provided with a through hole 6643 extending through a proximal end surface and a distal end surface thereof. The pushing member 682 can movably extend through the through hole 6643. The axis of the through hole 6643 coincides with the axis of the guide groove 6642. The proximal end surface of the rotating column 664 has two fixing holes 6646 provided on two opposite sides of the through hole 6643, and the distal ends of the two guide rails 651 are respectively fixed in the two fixing holes 6646.

As shown in FIGs. 48 to 50, the second rotating member 670 is arranged at the distal end of the housing 61 rotatable about the axis of the pushing member 682. The second rotating member 670 is a cylinder. A central part of the second rotating member 670 is axially provided with a through hole 6701, and the proximal end of the pushing tube 684 is fixed in the through hole 6701. The pushing member 682 movably extends through the through hole 6701. The axis of the through hole 6701 coincides with the axis of the pushing tube 684, and the axis of the second rotating member 670 coincides with the axis of the through hole 6701.

Specifically, the second rotating member 670 includes a cylindrical operating portion 6702, a rotating portion 6704 protruding from a central part of a proximal end surface of the operating portion 6702, and an annular protruding sheet 6705 protruding from a distal end surface of the operating portion 6702. A side wall of the operating portion 6702 is provided with anti-skip strips, which facilitate the rotation of the second rotating member 670 by fingers. The rotating portion 6704 is a cylinder. A side wall of the rotating portion 6704 is provided with two annular guide groove 6706 along its circumference. The two guide grooves 6706 correspond to the annular support sheets defined by the first support sheets 6112 of the first shell 611 and the fourth support sheets 6132 of the second shell 613. The axes of the two guide grooves 6706 coincide with the axis of the through hole 6701, and the second rotating member 670 can rotate along the guide groove 6706. The axis of the annular protruding sheet 6705 coincides with the axis of the through hole 6701. Two opposite engaging hooks 6707 are provided on the annular protruding sheet 6705 in the radial direction, and the two engaging hooks 6707 correspond to the two engaging blocks 6164 of the cover body 6160. An internal thread 6708 is provided on an inner wall of the through hole 6701 at the proximal end, and an external thread is provided on a side wall of the pushing tube 684 at the proximal end corresponding to the internal thread 6708.

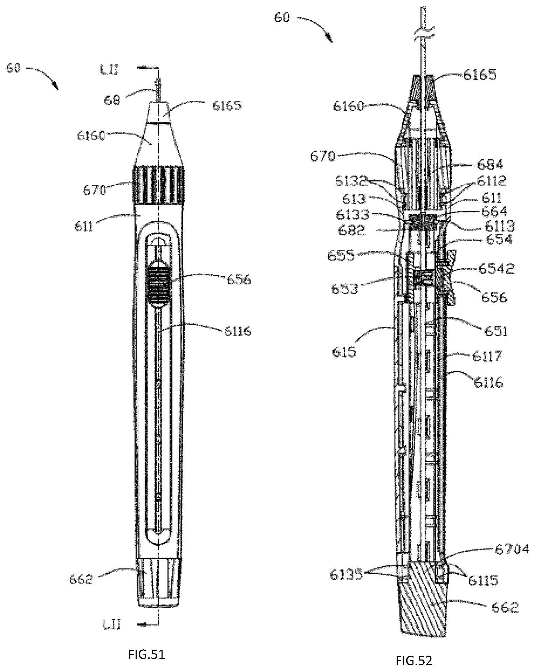

Referring also to FIGs. 29 to 52, when assembling the pushing device 60, the proximal end of the pushing member 682 movably extends through the through hole 6643 of the rotating post 664 and then is fixed in the fixing hole 6532 of the movable block 653. The two guide rails 651 slidably extend through the two through holes 6534 of the movable block 653 respectively, with the distal ends of the two guide rails 651 being fixed in the two fixing holes 6646 of the rotating post 664 respectively and the proximal ends of the two guide rails 651 being fixed in the two fixing holes 6626 of the first rotating member 662. The movable block 653 is slidably mounted around the guide rails 651 and located between the first rotating member 662 and the rotating post 664. The elastic members 658 are respectively mounted around the guide posts 6546. The movable block 653 is arranged between the first clamping block 654 and the second clamping block 655. The first clamping block 654 is fitted to the second clamping block 655, such that the movable block 653 is movably clamped in a receiving space defined by the first clamping groove 6548 of the first clamping block 654 and the second clamping groove 6558 of the second clamping block 655. The guide posts 6546 are slidably inserted into the corresponding guide holes 6554, and the elastic members 658 are elastically pressed between first clamping block 654 and second clamping block 655. The first clamping block 654 is moved close to the second clamping block 655 so that the guide plate 6547 extends into and is engaged in the access hole 6556, and is able to drive the second clamping block 655 to slide. The side wall of the guide plate 6547 abuts against the inner peripheral surface of the access hole 6556 without a gap between the guide plate 6547 and the access hole 6556, thereby preventing the first clamping block 655 from moving relative to the second clamping block 655 in the axial direction. As such, the movable block 653 can be driven to and positioned at a proper position more conveniently and accurately. The two guide rails 651 are located between the first shell 611 and the second shell 613 in such a manner that the connecting posts 6545 of the first clamping block 654 is opposite to the strip-shaped opening 6116 of the first shell 611. The second support sheet 6113 of the first shell 611 is inserted into the guide groove 6642 of the rotating post 664, and the two third support sheets 6115 are respectively inserted into the two guide grooves 6625 of the first rotating member 662. At this time, the sliding strip 6542 and the connecting post 6545 are both inserted into the strip-shaped opening 6116, the sliding strip 6542 can slide axially along the strip-shaped opening 6116, and the two sets of engaging teeth 6544 of the first clamping block 654 correspond to two racks 6117 respectively. The two connecting blocks 6564 of the button 656 are mounted around the two connecting posts 6545, and the recess 6561 of the button 656 exposes from the first shell 611. The button 656 can slide along the strip-shaped opening 6116.

The slipcover 6165 is fitted to the distal end of the cover body 6160. Specifically, the pair of protruding sheets 6167 of the slipcover 6165 are engaged to the engaging ring 6163 of the cover body 6160. The end cover 616 is engaged to the distal end of the second rotating member 670. Specifically, the annular protruding sheet 6705 is inserted into the annular groove 6162 of the cover body 6160 and the two engaging hooks 6707 are respectively engaged to the two engaging blocks 6164, such that the end cover 616 is fixed to the second rotating member 670. The axis of the through hole 6161 of the end cover 616 coincides with the axis of the through hole 6701 of the second rotating member 670. In other embodiments, the engaging hooks 6707 of the second rotating member 670 and the engaging blocks 6164 of the cover body 6160 can be omitted, and the annular protruding sheet 6705 of the second rotating member 670 is rotatably inserted into the annular groove 6162 of the cover body 6160, so that the end cover 616 is rotatably connected to the distal end of the second rotating member 670. The proximal end of the pushing tube 684 is caused to extend through the through hole 6161 of the end cover 616 and then fixed in the through hole 6701 of the second rotating member 670. Specifically, the external thread of the pushing tube 684 is screwed to the internal thread 6708 of the second rotating member 670. In other embodiments, the proximal end of the pushing tube 684 can be fixed in the through hole 6161 by welding, gluing or snap-fitting.

The distal end of the pushing member 682 is caused to extend through the through hole 6701 and the pushing tube 684 from the proximal end of the second rotating member 670, such that the second rotating member 670 is caused to slide to the distal end of the first shell 611. The two first support sheets 6112 of the first shell 611 are separately inserted into the two guide grooves 6706 of the second rotating member 670. The second shell 613 is connected to the first shell 611, and the engaging hooks 6131 of the second shell 613 are respectively engaged to the engaging pieces 6118 of the first shell 611. At this time, the two fourth support sheets 6132 of the second shell 613 are respectively inserted into the two guide grooves 6706 of the second rotating member 670, the fifth support sheet 6133 is received in the guide groove 6642 of the rotating post 664, the two sixth support sheets 6135 are respectively inserted into the two guide grooves 6625 of the first rotating member 662, the two rails 6136 are respectively received in the two chutes 6552 of the second clamping block 655, the elastic member 658 pushes the first clamping block 654 to cause the engaging teeth 6544 to be engaged to the racks 6117, and the operating portion 6702 of the second rotating member 670 is exposed from the housing 61. Then the outer buckle plate 615 is engaged into the mounting opening 6138 of the second shell 613. Specifically, the positioning posts 6151 of the outer buckle plate 615 are engaged into the engaging holes of the respective positioning blocks 6137, and the engaging hooks 6153 are engaged to the corresponding engaging holes 6139 such that the installation is completed.

When using the pushing device, the button 656 is pressed to move the first clamping block 654 towards the second clamping block 655. The elastic members 658 are resiliently deformed until the movable block 653 is clamped and positioned by the first clamping block 654 and the second clamping block 655. The engaging teeth 6544 of the first clamping block 654 are disconnected from the racks 6117. The button 656 is caused to slide towards the proximal end or the distal end along the strip-shaped opening 6116 of the first shell 611, so that the movable block 653 slides proximally or distally along with the first clamping block 654 and the second clamping block 655 to drive the pushing member 682 to slide proximally or distally in the pushing tube 684, thereby achieving the axial sliding of the pushing member 682. When the pushing member 682 slides to a proper position, the button 656 is released, and the elastic member 658 is elastically reset and pushes the first clamping block 654 to move away from the second clamping block 655 until the engaging teeth 6544 of the first clamping block 654 are engaged to the racks 6117. The sliding member 650 is positioned to prevent the movable block 653 from sliding in the axial direction, thereby positioning the pushing member 682 in the axial direction. When it is required to rotate the pushing member 682, the button 656 is released, to cause the first clamping block 654 to move away from the second clamping block 655. The first rotating member 662 is rotated to drive the guide rails 651, the movable block 653 and the rotating post 664 to rotate. That is, the movable block 653 can rotate in the clamping groove defined by the first clamping groove 6548 of the first clamping block 654 and the second clamping groove 6558 of the second clamping block 655 about the axis of the pushing member 682, thereby driving the pushing member 682 to rotate with respect to the pushing tube 684. When it is required to rotate the pushing tube 684, the operating portion 6702 of the second rotating member 670 is rotated to cause the second rotating member 670 to rotate about the axis of the pushing member 682. As the proximal end of the pushing tube 684 is fixed in the through hole 6701 of the second rotating member 670, the pushing tube 684 rotates with the second rotating member 670.

The sliding means 65 of the pushing device 60 can drive the pushing member 682 to slide in the axial direction, so as to facilitate changing the position of the occluder in the blood vessel which is fixed to the distal end of the pushing member 682, and control the release speed of the occluder. After the occluder is moved by the pushing member 682 to a proper position, the button 656 is released, and the first clamping block 654 is moved towards the first shell 611 by the resetting force of the elastic member 658, so that the engaging teeth 6544 are engaged to the racks 6117, to achieve the positioning of the pushing member 682 and the occluder. Therefore, the occluder can be conveniently moved and positioned by the sliding means 65.

In other embodiments, the first support sheet 6112 and the fourth support sheet 6132 may protrude from the outer peripheral surface of the rotating portion 6704 of the second rotating member 670, and the annular guide grooves 6706 may be defined on the inner walls of the first shell 611 and the second shell 613. The second support sheet 6113 and the fifth support sheet 6133 may protrude from the outer peripheral surface of the rotating post 664, and the guide grooves 6642 may be defined on the inner walls of the first shell 611 and the second shell 613. The third support sheet 6115 and the sixth support sheet 6135 may protrude from the outer peripheral surface of the rotating portion 6704, and the annular guide grooves 6706 may be formed on the inner walls of the first shell 611 and the second shell 613.

For the occluder fastening system, in an interventional operation, after a puncture position is determined and the puncture is completed, the distal end of the expansion rod 32 of the expander 30 is caused to extend into the sheath 23 from the proximal end of the sheath bendable adjustment device 20 through the proximal end cap 218, the seal ring 219 and the positioning tube 217, and be exposed from the distal end of the sheath. As the distal end of the expander 30 gradually decreases in diameter to form a conical structure, the sheath can be conveniently leaded to the vicinity of a lesion site by an operator under an imaging device with the aid of the distal structure of the expander 30. After reaching the vicinity of the lesion site, the expander 30 is withdrawn, with the sheath 23 left in the body to establish a passage from the outside to the body. The distal end of the pushing component 68 of the pushing device 60 is caused to extend through the sealing seat 44 and the loading tube 42 of the loading component 40. Then the distal end of the pushing member 682 and the distal end of the pushing tube 684 fitted each other are detachably fixed to the occluder 10. That is, the protrusion portion 152 and the pushing tube 684 are fixed by threaded connection. The connecting bolt 112 and the pushing member 682 are fixed by threaded connection. The relative position of the pushing member 682 and the pushing tube 684 is changed to pull the occluder 10 in the axial direction so that the occluder 10 can be easily received in the inner cavity of the loading tube 42 of the loading component 40. The distal end of the loading tube 42 is caused to extend into the sheath 23through the cap 218, the seal ring 219 and the positioning tube 217, to obtain a delivery system loaded with the occluder 10. The housing 61 of the pushing device 60 is held by a hand and the pushing component 68 is advanced distally, to deliver the occluder 10 to the distal end port of the sheath 23 of the sheath bendable adjustment device 20. By rotating the sheath bendable adjustment device 20 and bending the flexible section 235 of the sheath 23, the flexible section 235 of the sheath 23 is caused to face the rupture at the lesion site. The button 656 of the pushing device 60 is slowly pushed, to move the pushing member 682 to which the occluder 10 is detachably fixed towards the rupture to expose the occluder 10 from the sheath 23, and the occluder 10 is released to a proper position at the rupture. The button 656 of the pushing device 60 is slowly moved, to slowly move the pushing member 682 in the axial direction towards the proximal end, to make the first occluding portion 11 and the second occluding portion 15 of the occluder 10 be located at two ends of the rupture. Under the actions of the sliding means 65, the first rotating means 66 and the second rotating means 670 of the pushing device 60, the occlusion and locking of the rupture by the occluder 10, and the release of the occluder 10 from the pushing member 682 and the pushing tube 684 are achieved.

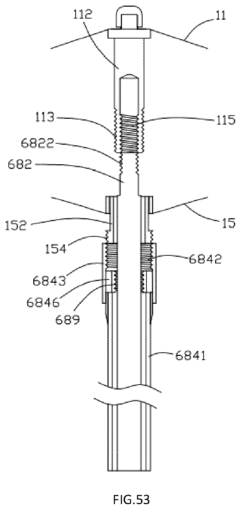

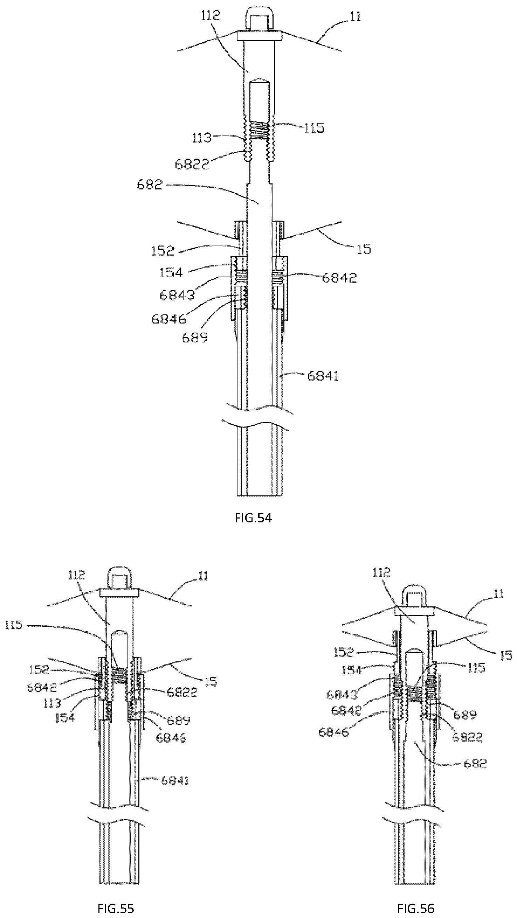

Referring to FIGs. 1 to 58, the fastening process of the occluder 10 in the occluder fastening system and the release process of the pushing member 682 and the pushing tube 684 from the occluder 10 in the first embodiment are described as follows.

Step 1: The nut 689 is engaged into the engaging groove 6846 of the extension section 6843, the third external thread 6822 of the pushing member 682 is fitted to the thread 115 of the connecting bolt 112, and the second internal thread 6842 of the pushing tube 684 is fitted to the second external thread 154 of the protrusion portion 152. Preferably, the protrusion portion 152 reaches the external thread at the proximal end of the pushing tube 684 and abuts against the engaging groove 6846.

Step 2: The button 656 is caused to slidably move towards the proximal end along the axial direction, and drives the pushing member 682 to slidably move towards the proximal end along the axial direction, such that the first external thread 113 of the connecting bolt 112 abuts against and is connected to the nut 689; the button 656 is released, and the elastic member 658 is elastically reset to push the first clamping block 654 to move radially, until the engaging teeth 6544 of the first clamping block 654 are engaged to the racks 6117, and thus the position of the connecting bolt 112 is fixed; the operating portion 6702 of the second rotating member 670 is rotated to cause the second rotating member 670 to rotate about the axial axis, and drive the pushing tube 684 to rotate so that the protrusion portion 152 is screwed out distally. Since the thread screwing direction between the pushing tube 684 and the protrusion portion 152 is opposite to the thread screwing direction between the connecting bolt 112 and the nut 689, the pushing tube 684 driving the nut 689 in the engaging groove 6846 to rotate together and thus the connecting bolt 112 is screwed towards the proximal end, and the connecting bolt 112 is tightly fastened to the nut 689. When the thread pitch and thread number of the second internal thread 6842 of the pushing tube 684 are the same as those of the nut 689, the nut 689 is completely mounted around the connecting bolt 112 when the protrusion portion 152 is screwed out of the pushing tube 684. When the thread pitch and thread number of the second internal thread 6842 of the pushing tube 684 are greater than those of the nut 689, the nut 689 is completely mounted around the connecting bolt 112 and the proximal end of the connecting bolt 112 is exposed from the proximal end of the nut 689 when the protrusion portion 152 is screwed out of the pushing tube 684.

Step 3: The button 656 is pressed to cause the engaging teeth 6544 to be released from the rack 6117, the button 656 is slidably moved distally in the axial direction to drive the pushing member 682 to slide distally in the axial direction, and the nut 689 is pushed out of the engaging groove 6846 and to abut against the proximal end surface of the protrusion portion 152. Preferably, it is determined whether the connecting bolt 112 is exposed from the proximal end of the nut 689 under observation by an imaging device. If not, the pushing member 682 is slidably moved proximally in the axial direction to drive the nut 689 to abut against the pushing tube 684 and move into the engaging groove 6846. The pushing tube 684 is rotated reversibly to fasten the pushing tube 684 and the protrusion portion 152, and cause the connecting bolt 112 to disengage from the nut 689 at the same time. Then the operation in Step 2 is performed until it is determined that the proximal end of the connecting bolt 112 is exposed from the nut 689 under observation by the imaging device. If it is determined that the connecting bolt 112 is exposed from the proximal end of the nut 689 under observation, the occluder 10 is completely fastened, and it can be determined that the thread of the nut 689 has been completely screwed into the connecting bolt 112 and good connection between the connecting bolt 112 and the nut 689 is formed. In this way, the risk of release of the nut 689 due to poor fixation between the connecting bolt 112 and the nut 689 caused by the fact that only part of the thread of the nut 689 is screwed into the connecting bolt 112 can be avoided. The first rotating member 662 is rotated to drive the pushing member 682 to rotate to release the pushing member 682 from the connecting bolt 112. As such, the release of the occluder 10 is finished.

It can be seen from the above three steps that the pushing tube 684 is threadedly connected to the protrusion portion 152 of the occluder 10. The connecting bolt 112 is provided with internal and external threads and the connecting bolt 112 is fixed to the pushing member 682 by threaded connection. The fastening of the occluder 10 only requires slidably moving the pushing member 682 proximally in the axial direction, to cause the first external thread 113 of the connecting bolt 112 to abut against the internal thread of the nut 689 which is engaged in the engaging groove 6846 of the pushing tube 684, and then rotating the pushing tube 684 to drive the nut 689 to be screwed into the connecting bolt 112 to complete the fastening of the occluder 10. As the thread screwing direction between the pushing tube 684 and the protrusion portion 152 is opposite to the thread screwing direction between the connecting bolt 112 and the nut 689, and the thread number and thread pitch in the extension section 6843 and the thread number and thread pitch in the protrusion portion 152 are equal to or greater than the thread number and thread pitch in the nut 689, when the nut 689 is screwed into the connecting bolt 112, the protrusion portion 152 of the occluder 10 is screwed out of the pushing tube 684 to realize the disconnection of the protrusion portion 152 from the pushing tube 684. Finally, the pushing member 682 is rotated to be released from the connecting bolt 112. In this way, the occluder 10 can be released from the pushing component 68. It is observed that whether the proximal end of the connecting bolt 112 is exposed from the nut 689 under an imaging device. The operation of fastening the pushing component 68 and the occluder 10 can be repeated several times, until the proximal end of the connecting bolt 112 is exposed from the nut 689 to ensure that the occluder 10 is fastened completely. It can be seen that the occluder fastening system has the advantages of simple operation, and high reliability in disconnection and fastening.

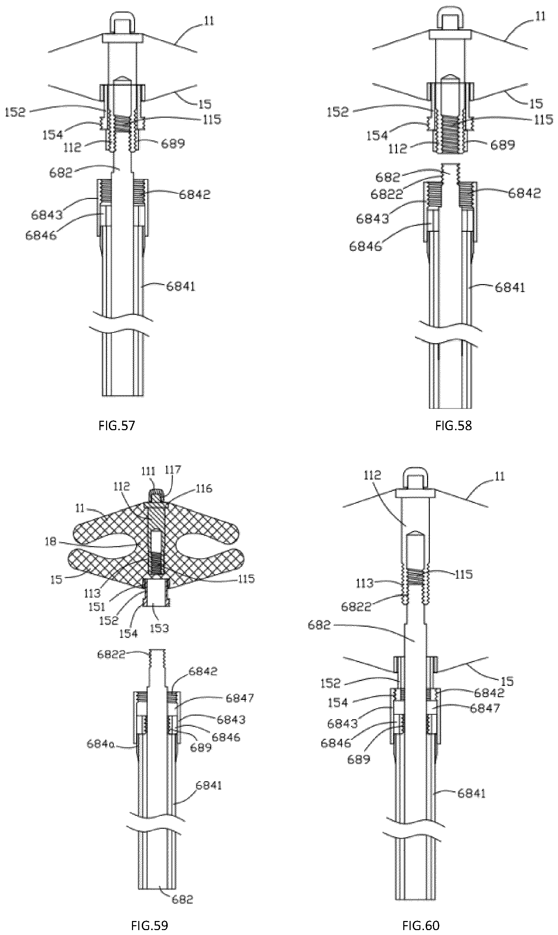

Referring to FIG. 59, the occluder fastening system provided in the second embodiment of the present application has a structure is similar to that of the first embodiment, the difference is in that the structure of the pushing tube 684a of the pushing device in the second embodiment is slightly different from that of the pushing tube 684 in the first embodiment. In the second embodiment, the inner wall of the extension section 6843 of the pushing tube 684a is provided with an idle groove 6847 between the second internal thread 6842 and the engaging groove 6846. When the protrusion portion 152 is connected to the pushing tube 684, the second external thread 154 of the protrusion portion 152 can rotate in the idle groove 6847. Specifically, the second internal thread 6842 of the extension section 6843 does not proximally extend to the engaging groove 6846. That is, there is no internal thread provided in the inner wall of the extension section 6843 at the end adjacent to the engaging groove 6846. The inner diameter of the idle groove 6847 is the same as the outer diameter of the protrusion portion 152, and the axial length of the idle groove 6847 is not less than the axial extension length of the second external thread 154 of the protrusion portion 152.

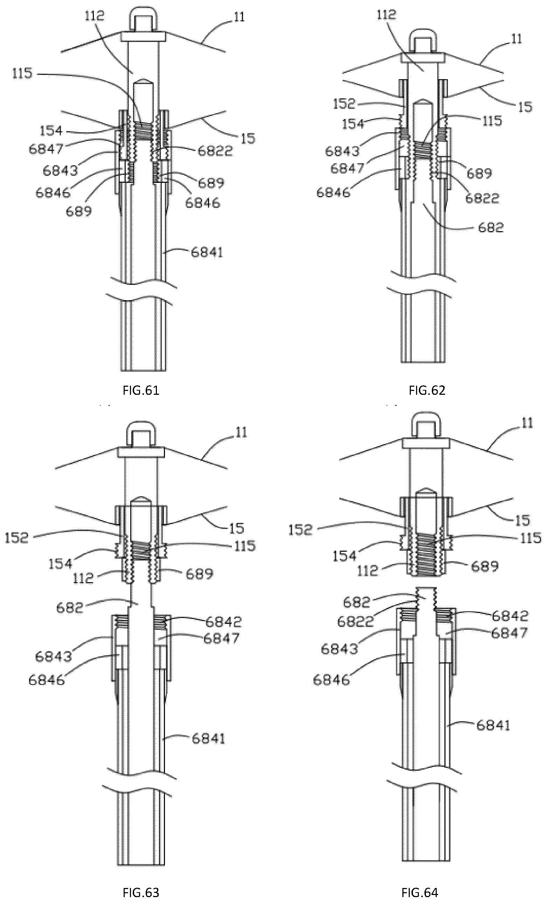

Referring to FIGs. 60 to 64, the fastening process of the occluder 10 in the occluder fastening system and the release process of the pushing member 682 and the pushing tube 684a from the occluder 10 in the second embodiment are described as follows.

Step 1: The nut 689 is engaged into the engaging groove 6846 of the extension section 6843, the third external thread 6822 of the pushing member 682 is fitted to the thread 115 of the connecting bolt 112, and the second external thread 154 of the protrusion portion 152 is fitted to the second internal thread 6842 of the pushing tube 684a, the pushing tube 684a is rotated to drive the protrusion portion 152 to move into the idle groove 6847 of the pushing tube 684. Preferably, the protrusion portion 152 reaches the external thread at the proximal end of the pushing tube 684a and abuts against the engaging groove 6846.

Step 2: The button 656 is slidably moved towards the proximal end along the axial direction, to drive the pushing member 682 to slidably move towards the proximal end along the axial direction, such that the first external thread 113 of the connecting bolt 112 abuts against and is connected to the internal thread of the nut 689; the button 656 is released, and the elastic member 658 is elastically reset to push the first clamping block 654 to move radially, until the engaging teeth 6544 of the first clamping block 654 are engaged to the racks 6117, such that the position of the connecting bolt 112 is fixed; and the operating portion 6702 of the second rotating member 670 is rotated continuously to cause the second rotating member 670 to rotate axially, and drive the pushing tube 684a to rotate continuously. The protrusion portion 152 is idling in the idle groove 6847, and thus the relative position of the connecting bolt 112 and nut 689 is adjustable, so that the first external thread 113 of the connecting bolt 112 completely abuts against the internal thread of the nut 689, and the connecting bolt 112 can be screwed smoothly in the nut 689. In this case, the pushing tube 684a drives the nut 689 in the engaging groove 6846 to rotate together and the connecting bolt 112 is screwed in proximally, and the connecting bolt 112 is fastened to the nut 689.

Step 3: The pushing member 682 is slidably moved distally in the axial direction, and the nut 689 is pushed out of the engaging groove 6846 and to abut against the proximal end surface of the protrusion portion 152. It is observed whether the connecting bolt 112 is exposed from the proximal end of the nut 689 under an imaging device. If not, the pushing member 682 is slidably moved proximally in the axial direction to drive the nut 689 to abut against the pushing tube 684. The pushing tube 684a is rotated reversibly to fasten the pushing tube 684a and the protrusion portion 152, and cause the connecting bolt 112 to disconnect from the nut 689 at the same time. Then Step 2 is performed until it is determined that the proximal end of the connecting bolt 112 is exposed from the nut 689 under observation through the imaging device. If it is observed that the connecting bolt 112 is exposed from the proximal end of the nut 689, the occluder 10 is completely fastened, and it can be determined that the thread of the nut 689 has been completely screwed into the connecting bolt 112 and thus a good connection between the connecting bolt 112 and the nut 689 is formed. In this way, the risk of release the nut 689 due to poor fixation of the connecting bolt 112 and the nut 689 caused by the fact that only part of the thread of the nut 689 is screwed into the connecting bolt 112 can be avoided. The first rotating member 662 is then rotated to drive the pushing member 682 to rotate to disconnect the pushing member 682 from the connecting bolt 112. In this way, the occluder 10 is detached.

From the fastening process of the occluder 10 in the occluder fastening system and the release process of the pushing member 682 and the pushing tube 684a from the occluder 10 in the second embodiment, it can be seen that since the idle groove 6847 is provided on the inner wall at the extension section 6843 of the pushing tube 684a between the second internal thread 6842 and the engaging groove 6846, in the process of adjusting the relative position of the connecting bolt 112 and the nut 689 to cause them to abut against each other, the protrusion portion 152 is idling in the idle groove. This is different from the situation in the occluder fastening system in the first embodiment where the protrusion portion 152 is rotating relative to the second internal thread 6842 in the extension section 6843, and is screwed out of the pushing tube 684. Because the sizes of the connecting bolt 112, the protrusion portion 152, the nut 689, and the engaging groove 6842 of the extension section 6843 are very small and usually in the range of several millimeters, there is inevitable dimension error existing in the connecting bolt 112, the nut 689, and the engaging groove 6864 manufactured by most of the commonly used manufacturing techniques. When the pushing member 682 is slidably moved proximally along the axial direction to cause the first external thread 113 of the connecting bolt 112 to abut against and be connected to the nut 689, the relative position where the connecting bolt 112 and the nut 689 abut against each other may be such that the connecting bolt 112 can be rightly screwed in the nut 689, or that the threads of the connecting bolt 112 and the nut 689 do not completely abut, and it requires to adjust the relative position of the connecting bolt 112 and the nut 689 to make the first external thread 113 of the connecting bolt 112 and the internal thread of the nut 689 completely abut, to thereby reach a position where the first external thread 113 can be screwed into the nut 689. Therefore, the idle groove 6847 provided in the extension section 6843 makes the interaction between the pushing tube 684a and the protrusion portion 152 almost negligible during the adjustment of the relative position of the connecting bolt 112 and the nut 689, which is much smaller than the friction force due to thread rotation between the pushing tube 684 and the protrusion portion 152 that are in threaded connection during the process of adjusting the relative position of the connecting bolt 112 and the nut 689 in the occluder fastening system in the first embodiment. On the one hand, this facilitates the adjustment of the relative position of the connecting bolt 112 and the nut 689 to reach a position where the connecting bolt 112 can be screwed into the nut 689. On the other hand, the internal thread abutting against the engaging groove 6846 provided in the extension section 6843 of the occluder fastening system in the first embodiment can also achieve the function of adjusting the relative position of the connecting bolt 112 and the nut 689 in the process of screwing the protrusion portion 152 out of the extension section 6843. However, this requires that the strength portion 152 has a sufficient length so that the protrusion portion 152 can be screwed and rotated in the extension section 6843 for a long time to adjust the relative position of the connecting bolt 112 and the nut 689. The longer protrusion portion 152 will remain at the position of the rupture after the interventional operation is completed, affecting the blood flow inside the peripheral blood vessels or the heart, and increasing the risk of postoperative complications. In the occluder fastening system in the second embodiment, as the idle groove 6847 is provided in the extension section 6843, in the process of adjusting the relative position of the connecting bolt 112 and the nut 689 to screw the connecting bolt 112 into the nut 689, the relative position of the protrusion portion 152 and the extension section 6843 does not change. This can greatly reduce the length of the protrusion portion 152, thereby reducing the length of the occluder 10 exposed out of the vascular wall or heart wall at the position of the rupture after the operation is completed, thereby significantly reducing the risk of postoperative complications.

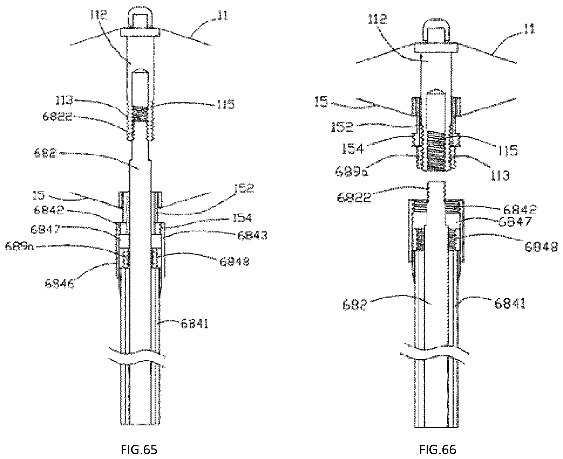

Referring to FIGs. 65 and 66, the structure of the occluder fastening system provided in the third embodiment of the present application is similar to that of the first embodiment, that the difference is in that a third internal thread 6848 is provided on the inner wall of the engaging groove 6846 which is located on the inner wall of the extension section 6843 at the proximal end in the third embodiment, the nut 689a is a cylindrical nut, the side wall of the nut 689a is provided with a fourth external thread, and the fourth external thread of the nut 689a can be screwed to the third internal thread 6848. The screwing direction between the fourth external thread of the nut 689a and the third internal thread 6848 is the same as the thread screwing direction between the second internal thread 6842 and the second external thread 154. The screwing direction between the fourth external thread of the nut 689a and the third internal thread 6848 is opposite to the screwing direction between the connecting bolt 112 and the internal thread of the nut 689a. Specifically, the engaging groove 6846 at the proximal end of the extension section 6843 is a circular hole, that is, the radial cross section of the engaging groove 6846 is circular, and the inner wall of the engaging groove 6846 is provided with the third internal thread 6848. The nut 689a is a cylindrical nut, the outer diameter of the nut 689a is the same as the inner diameter of the engaging groove 6846, and the side wall of the nut 689a is provided with the fourth external thread corresponding to the third internal thread 6848. The screwing direction between the third internal thread 6848 on the inner wall of the engaging groove 6846 and the fourth external thread of the nut 689a is the same as the screwing direction between the second internal thread 6842 of the extension section 6843 and the second external thread 154 of the protrusion portion 152. The screwing direction between the third internal thread 6848 on the inner wall of the engaging groove 6846 and the external thread of the nut 689a is opposite to the screwing direction between the first external thread 113 of the connecting bolt 112 and the internal thread of the nut 689a. Preferably, the second internal thread 6842 on the inner wall of the engaging groove 6846, the internal thread and external thread of the nut 689a, and the first external thread 113 of the connecting bolt 112 have the same thread pitch, number of turns, and overall thread length. During the process of rotating the pushing tube 684 to screw the nut 689a to the connecting bolt 112 to achieve the threaded connection of the nut 689a and the connecting bolt 112, the disengagement of the protrusion portion 152 from the extension section 6843 and the disengagement of the nut 689a from the engaging groove 6846 are also achieved in the meanwhile. In addition, instead of the engagement by regular shapes of a hexagon nut with the engaging groove 6846 that causes a dimensional error between the nut 689 and the engaging groove 6846, the threaded connection between the engaging groove 6846 and the nut 689a can greatly reduce the risk of requiring adjusting the relative position of the connecting bolt 112 and the nut 689a again when the pushing member 682 is slidably moved proximally in the axial direction to cause the first external thread 113 of the connecting bolt 112 to abut against and be connected to the internal thread of the cylindrical nut 689a. Therefore, the reliability in slidably moving the pushing member 682 proximal in the axial direction to cause the first external thread 113 of the connecting bolt 112 to abut against and be connected to the internal thread of the cylindrical nut 689a is increased, the step of adjusting the relative position of the connecting bolt 112 and the nut 689a is omitted, the length of the protrusion portion 152 is reduced, and the operation is simplified.

The fastening process of the occluder 10 in the occluder fastening system and the release process of the pushing member 682 and the pushing tube 684 from the occluder 10 in the third embodiment are similar to those in the first embodiment.

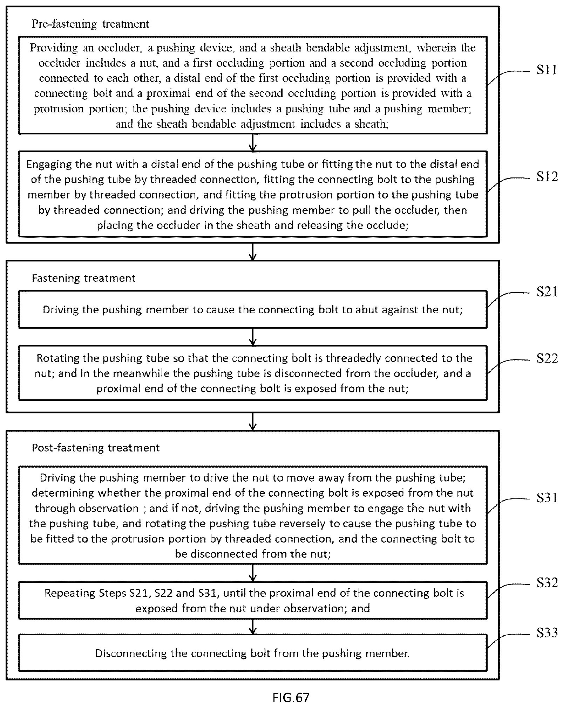

As shown in FIG. 67, the present application also provides a fastening method for an occluder fastening system, which includes a pre-fastening treatment, a fastening treatment and a post-fastening treatment. The pre-fastening treatment includes:

S11: providing an occluder 10, a pushing device 60, and a sheath bendable adjustment device 20, wherein the occluder 10 includes a nut 689, and a first occluding portion 11 and a second occluding portion 15 connected to each other, a distal end of the first occluding portion 11 is provided with a connecting bolt 112 and a protrusion portion 152 is provided at a proximal end of the second occluding portion 15; the pushing device 60 includes a pushing tube 684 and a pushing member 682; and the sheath bendable adjustment device 20 includes a sheath 23; and

S12: engaging the nut 689 with a distal end of the pushing tube 684 or fitting the nut 689 to the distal end of the pushing tube 684 by threaded connection, fitting the connecting bolt 112 to the pushing member 682 by threaded connection, fitting the protrusion portion 152 to the pushing tube 684 by threaded connection, and driving the pushing member 682 to pull the occluder 10, then placing the occluder 10 in the sheath and releasing the occluder 10.

The fastening treatment includes:
S21: driving the pushing member 682 to cause the connecting bolt 112 to abut against the nut 689; and
S22: rotating the pushing tube 684, so that the connecting bolt 112 is threadedly connected to the nut 689; in the meanwhile, the pushing tube 684 is disconnected from the occluder 10, and a proximal end of the connecting bolt 112 is exposed from the nut 689.

The post-fastening treatment includes:
S31: driving the pushing member 682 to drive the nut 689 to move away from the pushing tube 684; determining whether the proximal end of the connecting bolt 112 is exposed from the nut 689 through observation; if not, driving the pushing member 682 to make the nut 689 be engaged with the pushing tube 684, and rotating the pushing tube 684 reversely so that the pushing tube 684 is threadedly connected to the protrusion portion 152, and the connecting bolt 112 is disconnected from the nut 689 in the meanwhile;
S32: repeating Steps S21, S22 and S31, until the proximal end of the connecting bolt 112 is observed to be exposed from the nut 689; and
S33: disconnecting the connecting bolt 112 from the pushing member 682.

The fastening method for the occluder fastening system includes the following steps in practice:
The pre-fastening treatment includes:
S11: providing an occluder 10, a pushing device 60, and a sheath bendable adjustment device 20, wherein the occluder 10 includes a nut 689, and a first occluding portion 11 and a second occluding portion 15 connected to each other, a distal end of the first occluding portion 11 is provided with a connecting bolt 112 and a protrusion portion 152 is provided at a proximal end of the second occluding portion 15; the pushing device 60 includes a pushing tube 684 and a pushing member 682; and the sheath bendable adjustment device 20 includes a sheath 23; and establishing a passage through the sheath to the lesion site by skin puncture; and
S12: engaging the nut 689 with a distal end of the pushing tube 684 or fitting the nut 689 to the distal end of the pushing tube 684 by threaded connection, fitting the connecting bolt 112 to the pushing member 682 by threaded connection, and fitting the protrusion portion 152 to the pushing tube 684 by threaded connection; driving the pushing member 682 to pull the occluder 10, and then placing the occluder 10 in the sheath and releasing the occluder 10; and pushing by the pushing member 682, the first occluding portion 11 and the second occluding portion 15 of the occluder 10 are released and located at opposite ends of the rupture.

The fastening treatment includes:
S21: slidably moving the pushing member 682 to cause the first occluding portion 11 to approach the second occluding portion 15 and cause both the first occluding portion 11 and the second occluding portion 15 to be closely attached to the rupture, wherein at this time, the connecting bolt 112 abuts against the nut 689; and
S22: rotating the pushing tube 684, so that the connecting bolt 112 is threadedly connected to the nut 689; and in the meanwhile, the pushing tube 684 is disengaged from the occluder 10, and a proximal end of the connecting bolt 112 is exposed from the nut 689.

The post-fastening treatment includes:
S31: driving the pushing member 682 to drive the nut 689 to move away from the pushing tube 684; determining whether the proximal end of the connecting bolt 112 is exposed from the nut 689 through observation; and if not, driving the pushing member 682 to make the nut 689 be engaged with the pushing tube 684, and rotating the pushing tube 684 reversely so that the pushing tube 684 is threadedly connected to the protrusion portion 152, and the connecting bolt 112 is disconnected from the nut 689in the meanwhile;
S32: repeating Steps S21, S22 and S31, until the proximal end of the connecting bolt 112 is observed to be exposed from the nut 689; and
S33: rotating the pushing member 685, to disconnect the connecting bolt 112 from the pushing member 682. The pushing tube 684, the pushing member 682, and the sheath 23 are removed and the wound is sutured.

In an interventional operation, such a firm fastening manner by the connecting bolt 112 and the nut 689 is used for the occluder 10 in the fastening method of the occluder 10 provided in the present application. In the fastening process of fitting the connecting bolt 112 to the nut 689 by threaded connection, the pushing tube 684 is disengaged from the occluder 10, and the proximal end of the connecting bolt 112 is exposed from the nut 689. On the one hand, the adverse effect on the fastened structure of the occluder 10 is avoided during the process of release the pushing member 682 and the pushing tube 684 from the occluder 10. On the other hand, because the proximal end of the connecting bolt 112 is exposed from the nut 689, which structure can be observed under an imaging device. If the structure is not observed, it means that the occluder 10 is not completely fastened. At this time, the operation to the pushing member 682 and the pushing tube 684 may be repeated several times, to fasten with the occluder 10 again, until the proximal end of the connecting bolt 112 is exposed from the nut 689 to make sure that the occluder 10 is completely fastened. Therefore, the fastening method of the occluder 10 provided in the present application has the advantages of simple operation and reliable fastening.

The foregoing descriptions are embodiments of the present invention. It should be appreciated that some improvements and modifications can be made by those of ordinary skill in the art without departing from the spirit of the embodiments of the present invention, which should also be within the scope of the present application.

## Claims

1. An occluder, comprising a first occluding portion and a second occluding portion, **characterized in that**, a distal end of the first occluding portion is provided with a connecting bolt extending towards the second occluding portion, and a first external thread is provided on a side wall of the connecting bolt; a protrusion portion is provided at a proximal end of the second occluding portion, a through hole is provided in the protrusion portion, and a second external thread is provided on a side wall of the protrusion portion; a thread direction of the first external thread is opposite to a thread direction of the second external thread, and a proximal end of the connecting bolt is configured to extend through the through hole and is screwed to a nut.

2. The occluder according to claim 1, wherein the first external thread is a left-handed thread or a right-handed thread, the second external thread is a right-handed thread or a left-handed thread, and an effective thread length of the connecting bolt is greater than or equal to an effective thread length of the nut.

3. The occluder according to claim 1, wherein a screw hole extending distally is provided at a proximal end of the connecting bolt, and the screw hole is configured to be detachably connected to a distal end of a pushing member.

4. The occluder according to claim 3, wherein a first internal thread is provided on an inner wall of the screw hole, a third external thread is provided on the distal end of the pushing member, and the first internal thread is threadedly connected to the third external thread.

5. The occluder according to claim 1, wherein the first occluding portion and the second occluding portion are both woven with metal wires and have a two-layer disc-shaped arc-surface structure with an internal space, and the connecting bolt is located inside the internal space.

6. The occluder according to claim 5, wherein a positioning disc protruding radially is provided at a distal end of the connecting bolt.

7. The occluder according to claim 5, wherein the proximal end of the second occluding portion is provided with an inner sleeve, and the inner sleeve is located inside the second occluding portion; a distal end of the protrusion portion extends into the second occluding portion; and a proximal end of the metal wire is bent inward and then positioned between the inner sleeve and the protrusion portion.

8. An occluder fastening system, comprising a pushing device and an occluder according to any one of claims 1 to 7, wherein the pushing device comprises a first control component, a second control component and a pushing component, the pushing component comprises a pushing tube and a pushing member mounted inside the pushing tube; an inner wall of the pushing tube at a distal end is provided with a second internal thread corresponding to the second external thread; the first control component is capable of driving the pushing member to slide back and forth to drive the proximal end of the connecting bolt which is detachably connected to the pushing member to abut against or move away from the nut; the first control component is capable of driving the pushing tube to rotate, the second control component is capable of driving the pushing tube to rotate; and a screwing direction between the first external thread and the nut is opposite to a screwing direction between the projection portion and the pushing tube.

9. The occluder fastening system according to claim 8, wherein the pushing tube comprises a body section and an extension section arranged at a distal end of the body section; the nut is capable of being engaged in or disengaged from the extension section; the second internal thread is provided on an inner wall of the extension section; and the pushing member is capable of extending through the nut and the extension section.

10. The occluder fastening system according to claim 9, wherein an inner diameter of the extension section is larger than an inner diameter of the body section, and the nut abuts against the distal end of the body section.

11. The occluder fastening system according to claim 9, wherein an engaging groove is provided on an inner wall of the extension section at a proximal end thereof, and the nut is capable of being engaged in or disengaged from the engaging groove.

12. The occluder fastening system according to claim 11, wherein an idle groove is provided on the inner wall of the extension section between the second internal thread and the engaging groove, and the second external thread of the protrusion portion is capable of rotating in the idle groove.

13. The occluder fastening system according to claim 12, wherein an axial length of the idle groove is not less than an axial extension length of the second external thread of the protrusion portion.

14. The occluder fastening system according to claim 11, wherein a third internal thread is provided on an inner wall of the engaging groove, the nut is cylindrical, a fourth external thread is provided on a side wall of the nut, and the fourth external thread of the nut is connectable to the third internal thread by threaded connection, wherein a screwing direction between the fourth external thread and the third internal thread is the same as a screwing direction between the second internal thread and the second external thread, and the screwing direction between the fourth external thread and the third internal thread is opposite to a screwing direction between the first external thread and the nut.

15. The occluder fastening system according to claim 8, wherein the first control component comprises a sliding means and the sliding means is capable of driving the pushing member to slide back and forth in the pushing tube; the second control component comprises a second rotating means, and the second rotating means is capable of driving the pushing tube to rotate, to cause the connecting bolt to be screwed to the nut while causing the second external thread of the protrusion portion to be disengaged from the second internal thread of the pushing tube.

16. The occluder fastening system according to claim 15, wherein the sliding means comprises a guide rail extending in an axial direction and arranged in a housing, a movable block arranged around the guide rail, and a sliding member for driving the movable block to slide along the guide rail, wherein a proximal end of the pushing member is fixed to the movable block, and the movable block is capable of driving the pushing member to slide when the movable block slides under driving of the sliding member.

17. The occluder fastening system according to claim 16, wherein the sliding member comprises a first clamping block and a second clamping block, and the first clamping block is capable of move close to or away from the second clamping block; opposing side surfaces of the first clamping block and the second clamping block are recessed to form a clamping groove, and the movable block is rotatably engaged in the clamping groove; an elastic member is provided between the first clamping block and the second clamping block, and the elastic member is capable of driving the first clamping block to move away from the second clamping block.

18. The occluder fastening system according to claim 17, wherein an inner wall of the housing is provided with a strip-shaped opening, at least one side of the strip-shaped opening is provided with a rack, and the first clamping block is provided with an engaging tooth corresponding to the inner wall of the strip-shaped opening, wherein the elastic member is capable of elastically pushing the first clamping block to cause the engaging tooth to be engaged in the rack to lock the pushing member, and the first clamping block is capable of being pushed towards the second clamping block to release the engaging tooth from the rack so that the sliding member is capable of driving the movable block to slide.

19. The occluder fastening system according to claim 17, wherein an inner wall of the first clamping block facing the movable block is provided with a first clamping groove, an inner wall of the second clamping block facing the movable block is provided with a second clamping groove, the first clamping groove and the second clamping groove cooperatively define the clamping groove for accommodating the movable block, the movable block is engaged in the clamping groove in the axial direction, and the sliding member is capable of being driven to move, which in turn drives the movable block to slide.

20. The occluder fastening system according to claim 17, wherein the second clamping block is provided with a guide hole, the first clamping block is provided with a guide post that is extendable into the guide hole, and the elastic member is a spring mounted around the guide post, the first clamping block is movable close to or away from the second clamping block along the guide post; and the first clamping block is further provided with a guide plate, the second clamping block is provided with an access hole, and the first clamping block is movable close to the second clamping block so that the guide plate extends into the access hole, and is engaged to and drives the second clamping block to slide.

21. The occluder fastening system according to claim 17, wherein a side wall of the second clamping block close to the housing is axially provided with a chute, the housing is provided with a rail at a position corresponding to the chute, and the second clamping block is slidable along the rail.

22. The occluder fastening system according to claim 15, wherein the second rotating means comprises a second rotating member rotatably arranged at a distal end of the housing, a through hole is axially formed in a central part of the second rotating member, and the second rotating member and the pushing tube are threadedly fixed in the through hole.

23. The occluder fastening system according to claim 8, further comprising a sheath bendable adjustment device, the sheath bendable adjustment device comprising a sheath and an adjustment means, wherein the sheath comprises a bendable distal end, and the adjustment means comprises an adjustment member, a driving member for driving the adjustment member to move in the axial direction, and two pull wires, wherein distal ends of the two pull wires are slidably threaded through in a side wall of the sheath at different positions in the axial direction and are respectively connected to a distal end of the sheath; a wire winding portion is provided in a casing, wherein a proximal end of one of the pull wires is connected to the adjustment member, and a proximal end of an other one of the pull wires is wound around the wire winding portion and then connected to the adjustment member; and the driving member is capable of driving the adjustment member to move and drive the two pull wires to slide to bend the bendable distal end in different directions.

24. The occluder fastening system according to claim 23, wherein bending facilitating grooves are provided at positions on a side wall in a flexible section of the sheath corresponding to the pull wires.

25. A fastening method for an occluder fastening system, comprising a pre-fastening treatment, a fastening treatment and a post-fastening treatment, wherein the pre-fastening treatment comprises:
S11: providing an occluder, a pushing device, and a sheath bendable adjustment device, wherein the occluder comprises a nut, and a first occluding portion and a second occluding portion connected to each other, a distal end of the first occluding portion is provided with a connecting bolt and a proximal end of the second occluding portion is provided with a protrusion portion; the pushing device comprises a pushing tube and a pushing member; and the sheath bendable adjustment device comprises a sheath; and
S12: engaging the nut with a distal end of the pushing tube or fitting the nut to the distal end of the pushing tube by threaded connection, fitting the connecting bolt to the pushing member by threaded connection, fitting the protrusion portion to the pushing tube by threaded connection; and driving the pushing member to pull the occluder, then placing the occluder in the sheath and releasing the occluder; and
the fastening treatment comprises:
S21: driving the pushing member to cause the connecting bolt to abut against the nut; and
S22: rotating the pushing tube so that the connecting bolt is threadedly connected to the nut, and in the meanwhile the pushing tube is disconnected from the occluder, and exposing a proximal end of the connecting bolt from the nut.

26. The method according to claim 25, wherein the post-fastening treatment comprises:
S31: driving the pushing member to drive the nut to move away from the pushing tube; determining whether the proximal end of the connecting bolt is exposed from the nut through observation; and if not, driving the pushing member to engage the nut with the pushing tube, and rotating the pushing tube reversely to cause the pushing tube to be fitted to the protrusion portion by threaded connection, upon which the connecting bolt is disconnected from the nut;
S32: repeating Steps S21, S22 and S31, until the proximal end of the connecting bolt is exposed from the nut under observation; and
S33: disconnecting the connecting bolt from the pushing member.
